# EUROPEAN PATENT SPECIFICATION

(11) **EP 2 569 102 B1**
(45) Date of publication and mention of the grant of the patent: **11.08.2021**
(21) Application number: 11727752.5
(22) Date of filing: 10.05.2011
(51) Int. Cl.: B09B 3/00, B07B 1/20, B07B 1/22, B03B 9/06, A61L 11/00, B01F 7/00

(54) **SYSTEM FOR TREATMENT OF MUNICIPAL SOLID WASTE**
SYSTEM ZUR AUFBEREITUNG VON HAUSMÜLL
SYSTÈME DE TRAITEMENT DE DÉCHETS SOLIDES MUNICIPAUX

(30) Priority: 10.05.2010 GR 20100100267
(43) Date of publication of application: 20.03.2013
(73) Proprietor: SR Technologies (SRT) Ltd., 1111 Sofia (BG)
(72) Inventor: Soukou, Eleftheria, 222 Larisa (GR)
(86) International application number: PCT/GR2011/000019
(87) International publication number: WO 2011/141757

(56) References cited:
- EP-A2- 0 995 502
- EP-A2- 0 995 502
- WO-A1-2004/028715
- WO-A1-2004/028715
- WO-A2-2008/065452
- WO-A2-2008/065452
- CN-Y- 201 094 752
- CN-Y- 201 094 752
- US-A1- 2002 096 796
- US-A1- 2002 096 796

## Description

### FIELD OF THE INVENTION

The disclosure comes under the sector of mechanical engineering and refers to a cold method of treatment of municipal solid waste. It also refers to a system of apparatus applying this method.

### BACKGROUND OF THE INVENTION

The following types of waste belong to the category of municipal solid waste:
The biodegradable waste, such as food, the "green" waste (e.g. plants), etc.
The recyclable materials, such as paper, glass, aluminium and tin packaging, some types of plastic etc.
The inert materials, such as construction materials, gathered in large containers for their disposal, etc.
The composite materials, such as clothing, types of plastic toys, etc.
The so called "dangerous household waste", such as medicine, the electronic waste, paints, household sprays, the fertilizer and pesticide packaging, the shoes shiners etc.

The management of the municipal solid wastes has become a major problem of all the advanced societies, given that the contemporary way of living, characterized by the mass increase of the living standard of citizens, is considered that it amounts today to the creation of around 1 kg of municipal solid wastes per resident in daily basis.

In order to dispose the Municipal Solid Waste under the known treatment methods of today, they are first collected by the garbage trucks and then they are gathered to the Waste Transportation Stations.

Some of the main methods used today for the management of the municipal solid waste are:
The mechanical treatment, the anaerobic treatment of a biodegradable fraction of the waste for the production of biogas and recycling, the thermal methods of treatment (such as burning, sterilization, cracking, incineration for energy recovery, plasma, etc.), the biological methods of treatment like the biological drying that produces a stabilized product destined for sanitary landfills, the mechanical selection and compost production - the anaerobic fermentation, etc., and finally landfills.

Materials recycling methods from waste necessitate special infrastructure, which is nonexistent in developing countries. Apart from that, it cannot be applied to all the municipal waste, but only to the recyclable materials, such as paper, glass, aluminum and some plastics and it also presents a high cost.

The energy recovery method and the landfills of wastes are two methods, that their application involve many negative consequences, with high risk for public health, like emissions of high heavy metals concentrations, and generally emission of gas, liquid, and solid wastes, ground pollution from heavy metals, emissions of intense odors that attract any type of animals and rodents.

Wherever these places are created the whole surrounding area is degraded, the high cost of their application must also be included. Because a complete system of municipal waste management also includes the application of trans-shipment systems for the increase of the financial efficiency of the system. Furthermore, in many countries; and in the European Union as well, a legal framework is in force, which provides for the admission of landfills of waste only if they have been submitted to special treatment in advance.

Therefore, a complete system for the management of municipal waste includes, before the disposal of the final waste to modern landfills, the use of treatment methods aiming to the energy exploitation or the re-use of the materials. Such treatment methods are the natural, thermal, chemical or biological treatment.

Furthermore, one of the biggest disadvantages of the above treatment methods is the fact that their application necessitates the consumption of enormous quantities of energy. The immense energy need of all the above methods makes them extremely expensive with a high cost as much for the economy of a country but also for the environment.

WO 2004/028715 discloses a system for treatment of Municipal Solid Waste in accordance with the preamble of claim 1, which is comprising a reception funnel for entry of the Municipal Solid Waste into the system, a Special Opening Unit and a Special Homogenization Unit. The system is comprising a reception funnel for entry of the Municipal Solid Waste into the system, under which there is a first conveyor for transfer of the waste towards a Special Opening Unit of the Municipal Solid Waste packages and a Special Homogenization Unit of the Municipal Solid Waste. Outside the reception funnel, there is at least one separation system. The system is comprising also a vertical pad elevator, a Measuring Feeder for controlling the feeding flow of a Low Frequency Pulsator for pulverization of material and a High Frequency Pulsator. An Air Transfer system is configured to transfer the product from the High Frequency Pulsator to a Stabilization Chamber.

Because of all the above disadvantages present in the most common municipal solid waste management methods applied today, it is imperative for modern societies to seek other solutions for their essential management.

### SUMMARY OF THE INVENTION

The present disclosure constitutes a cold and not a thermal management of municipal solid waste and is not submitted to the existing technical level regarding the methods of Municipal Solid Waste management. Its application produces a final compressed dehydrated and solidified product from the waste in various forms, equal to 1/10 of the initial volume of the waste submitted to the treatment. The form of the final products can be in pellets, flakes, powder, spherical, of small or big granulometry etc. The final product has a particularly high thermal power and is adequate for many uses. The invention is defined by the system for treatment of Municipal Solid Waste in accordance with claim 1.

The municipal wastes (organic or not) are submitted to the present method in the condition they are after their collection by the garbage trucks or after their concentration in areas of waste trans-shipment, the way they are in the garbage bags without a pre-treatment being necessary.

A summarized description of the stages of the method is following along with a system of apparatus applying it. The method includes seven obligatory steps, and four optional ones:
1^{st} Step: Entrance of the waste into the system;
2^{nd} Step: Treatment of opening and cutting by using the special opening unit;
3^{rd} Step: Homogenization treatment by using the special homogenization unit;
4^{th} Step (optional): First separation of the ferrous metallic elements with magnetic elements;
5^{th} Step: Treatment of first pulverization by using a low frequency pulsator;
6^{th} Step: Further pulverization treatment by using a high frequency pulsator;
7^{th} Step: Exposure to an electric field;
8^{th} Step: Exposure to UV radiation emission;
9^{th} Step (optional): Separation of glass, ferrous metallic elements, aluminium and plastic, by using a sorter of recyclable materials and channeling of the selected materials to recycling;
10^{th} Step (optional): Exposure of the lighter elements to microwave radiation;
11^{th} Step (optional): Array of final stabilization, volume and mass decrease (Pelletizing).

### 1^{st} Step: Entrance of the waste into the system

Initially the wastes, without being submitted to any treatment, are simply thrown inside the reception funnel in any possible way. On the lower part of the reception funnel there is one first chain conveyor (or, alternatively, a conveyor belt), which transports the waste to the next stage. Also, at the bottom part of the reception funnel and under the first chain conveyor, there is a drainage collection and separation system.

### 2^{nd} Step: Treatment of opening and cutting by using the special opening unit.

In the following stage the wastes as they are still closed inside bags and after a visual control by an electronic eye or/and CCD cameras in order to detect possible movement or big objects that may create problems to the operation of the system, the packaging is opened by shredding. The system that applies this method possesses a Special Opening Unit (19) and the visual control device (20).

### 3^{rd} Step: Homogenization treatment by using the special homogenization unit.

In the following stage the shredded wastes are cut to smaller pieces, and in this way they are homogenized. The system that applies this method possesses for this reason a Special Homogenization Unit (28).

Then the product is transferred by a horizontal conveyor (136) to a vertical pad elevator (31) and from there to the next stage of treatment. The horizontal conveyor (136) is used in the case that a selection of recyclable materials is desired at this stage. In the case that the selection of the recyclable materials is not desired at this stage, then the use of the horizontal conveyor (136) is not necessary and the product goes directly to the vertical pad elevator (31) and from there to the horizontal Measuring Feeder (38) for the 4^{th} step to take place.

### 4^{th} Step (optional): First separation of the ferrous metallic elements by magnetic elements.

At this point a first optional separation/ selection of the ferrous metallic ground parts of the waste can take place, and in particular of the larger ones with the use of magnetic elements. The system that applies this method possesses for this reason a special Measuring Feeder (38).

### 5^{th} Step: Treatment of first pulverization by using a low frequency pulsator

On the following stage, the wastes with homogenized form are submitted to a procedure of fast rotation and percussion, until they become very small in size. The system that applies this method possesses for this reason a first Low Frequency Pulsator.

### 6^{th} Step: Further pulverization Treatment by using a high frequency pulsator.

In the following stage the outgoing product, in the small size that it has now, is transferred again by a second chain conveyor and is submitted to the same or even faster rotation and percussion, until it becomes even smaller. At this stage, the percussion takes place by pulses of higher frequency than in the previous step. The system that applies this method possesses for this reason a special second Pulsator of High Frequency.

### 7^{th} Step: Exposure to an electric field.

In the following stage the outgoing product has now acquired a fibrous form and is transferred with an air transfer system - to a special Stabilization Chamber with a screw and stirrer and a system of moisture regulation of the product and removal - recovery arrays of the recyclable products. The product is placed within an electric field. Because of the materials the product consists of, but also because of its moisture, which can be regulated, an instantaneous jumping of electrons takes place resulting to the sterilization of the mass of the product inside the Chamber. At the same time, the air transfer process causes violent dehumidification of the outgoing product and consequently the removal of unpleasant odors and partially the extermination of the pathogenic microorganisms and its sterilization.

### 8^{th} Step: Exposure to UV radiation emission.

In the following stage, the product remaining within the Stabilization Chamber is submitted to exposure of UV radiation.

### 9^{th} Step (optional): Separation of glass, ferrous metallic elements, aluminium and plastic, by using a sorter of recyclable materials and channeling materials to recycling.

Three separations of elements of the product are carried out at the same time and depending on their granulometry - they are inserted to the Special Sorter by respective openings of three filters. In this way the removal of the recyclable materials is accomplished which are following separated and guided for recycling. The majority of the elements guided to selection and recycling are glass, ferrous metallic elements, aluminium and plastic. Given that this stage is optional, these elements can be removed in bulk to be led to special outside selection units of recyclable materials or they can be led to the Special Sorter of Recyclable Materials that the system applying the invention has, which effectuates the selection of the elements, some by gravity, some by magnetic elements, some with a flow of air, and some with the use of electrostatic electricity.

### 10^{th} Step (optional): Exposure of the lighter elements to microwave radiation.

In the following stage the products are placed in conditions of microwave exposure which have as a result the additional sterilization of the outgoing mass. The system that applies the method possesses for this reason a special Chamber of Microwave Radiation.

### 11^{th} Step (optional): Array of final stabilization, volume and mass decrease (Pelletizing)

In the final stage the product passes through a compression machine (pellet machine) from which we take the final dehydrated and stabilized product which is led to the packaging machine. For this reason the system that applies this method possesses a final Stabilization layout, volume and mass decrease - a pellet machine. The product, during its stay at this Layout is submitted to the proper pressure and temperature conditions. In this way the final stabilization of the outgoing product is accomplished.

The system of machines that applies this method possesses in many points visual control systems, which have the capacity to stop the operation of the system whenever it is needed (e.g. in order to remove the structural material of large dimensions like iron bars etc., or some organism like animals for example).

The method and the system of machines that apply it are a continuous operation system with the sense that the product (the Municipal Solid Waste), enters in its initial condition and comes out directly to the final, without time consuming procedures and in the minimum period of time.

The whole operation of the system is controlled by a Control and Handling Unit from where the user/s can supervise and control every phase of the procedure and every subsystem. The Control and Handling Unit is equipped with screens, computers, manual controls and anything else considered necessary for the supervision and control of the procedure.

Where there is no possibility for the supply of electricity from the network, the system can be supplied with electricity by a power generator.

This method is applied to all the municipal wastes (organic or not), which after the application of the method are in an initial dehydration condition, while next, in this dehydrated state, the material is inserted to the pelletization machine and comes out stabilized and compressed as Pellets. The result of the application of this method is the extraction approximately of 1/10 of the initial product, that is, 1000 liters of waste give approximately 100 liters of the final product.

A first advantage presented by the present method is that initially it constitutes a completely ecological method of MSW treatment, as it does not apply thermal treatment methods (like burning or pyrolisis), it does not use heat generating sources, it does not produce emission of air pollutants in the atmosphere, or the creation of dangerous solid and liquid wastes. All the applied methods of MSW treatment until today use some of the above means. On the contrary, the present method suspends landfills and the classic burning, and therefore it does not present the risks for the environment and humans presented by the above methods.

Another advantage of the present method is that it transforms the MSW to an inert, namely sterilized material, free of the infectious pathogenic factors that the wastes have. This advantage is extended by the fact that the elimination of the infectious load results as a natural phenomenon by this method, of the decrease of the volume of the waste, without the use of chemicals, without burning or burying. The MSW which is submitted to the treatment of the present method, at the initiation of the method is in its initial state and at the end at a dehydrated state, having lost a percentage of 90% of its initial volume.

Another advantage of the present method is that it can equally be applied to all the Municipal Solid Waste, organic or not without a special treatment being needed.

Also, an advantage of the present method is that we can, at specific stages of the treatment, recover recyclable materials, like ferrous and non ferrous metals, glass, plastic, existing in the waste during its entry in the system.

Another advantage of the present method is that its application permits to the relatively competent entities (in most countries are the Local Administration Organizations) to effectuate the MSW treatment in a much more economical way. This is because, the application of the present method does not require the expenses needed today for the transfer of the solid waste to remote locations for their treatment or for the transportation of this waste to the landfills. These are the expenses for personnel, purchase for transportation means (like trucks and lifting machines), cleaning expenses, maintenance, etc.

Furthermore, the wastes are transformed directly to an industrial mass admissible and not dangerous.

A relevant advantage of this fact is that the application of the present method does not aggravate further the environment, since it consists an environmentally extremely safe way of MSW treatment, the coming and going of the trucks for the transportation of waste to other locations or to the landfills is decreased and therefore the emissions of the air pollutants generated by the transportation trucks are also decreased. Also, the present method makes landfills redundant and eliminates them. This results to a high profit as much for the environment, since the MSW do not remain in these areas and the aquifer is not polluted, as for the financial profit for the entities that use today the landfills, by not paying anymore the fees paid today for the use of these areas.

Another advantage of the present method is that the mechanical system that applies it, that is the mechanical equipment, accompanied by the necessary electrical and electronic equipment, can be constructed in such dimensions to fit inside containers, like transport containers. These containers, (which can also have cooling units), can be transported by a train, ship, truck, they can be transported directly to the Waste Transportation Stations of the local administration organizations and apply on site the present waste treatment method and also to the landfills where the waste is buried today. The possibility of placement of the mechanical system of this invention within a container makes the invention flexible, transportable and applicable in any way.

An advantage of the present invention is the fact that regarding the existing methods it necessitates smaller energy consumption for its application.

### BRIEF DESCRIPTION OF THE DRAWINGS

The attached drawings present the following in brief:
Drawing 1 presents in total three containers in which the total of the electromechanical equipment has been placed applying the present method. It also depicts a loader inserting waste in the system in the reception funnel (1). It also depicts the sheets (2) of the reception funnel (1), the vertical pad elevator (31), the horizontal measuring feeder (38) and the control and handling unit of the system (129).
Drawing 2 presents a view of part of the machines' system that apply the present method and in particular the reception funnel (1), the drainage tank (15) and the pump (16) for thick liquids flowing from the waste and from the access manholes (26), as well as the special opening and homogenization units (19, 28).
Drawing 3 presents the reception funnel (1) of the wastes with a detailed depiction of its components and the self-cleaning separation system it has (17).
Drawing 4 presents the reception funnel of the wastes from the front, with the first chain conveyor (5).
Drawing 5 presents the first chain conveyor (5) in detailed depiction.
Drawing 6 presents the interior arrays of the Special Opening Unit (19) and of the Homogenization Unit (28).
Drawing 7 presents analytically the interior of the Special Opening Unit (19) and the circular cutting disks (22) it has.
Drawing 8 presents the interior of the Special Homogenization Unit (28) with the component of the rotating axis (29) and the multiple metallic blades (30) it has.
Drawing 9 presents the vertical pad elevator (31), the horizontal measuring feeder (38) and the -of optional use- horizontal conveyor (136).
Drawing 10 presents the horizontal measuring feeder (38) with the rotating axis (41) and the magnetic elements (140) it has as an option.
Drawing 11 presents almost the total of the mechanical system applying the present method and mainly the Low Frequency Pulsator (39), the second chain conveyor (66), the High Frequency Pulsator (68) and the Stabilization Chamber (83).
Drawing 12 presents the -optional- Special Sorter of Recyclable Materials (122) and the metallic filters (120, 121, 141).
Drawing 13 presents the second chain conveyor (66) of waste transportation.
Drawing 14 presents in detail the technical elements of the interior of the Special Sorter of Recyclable Materials (122) (optional).
Drawing 15 presents the Stabilization Chamber (83).
Drawing 16 presents the interior of the Stabilization Chamber (83) with the screw (103) and the stirrer (104).
Drawing 17 presents the interior of the Low Frequency Pulsator (39).
Drawing 18 presents the interior of the High Frequency Pulsator (68).

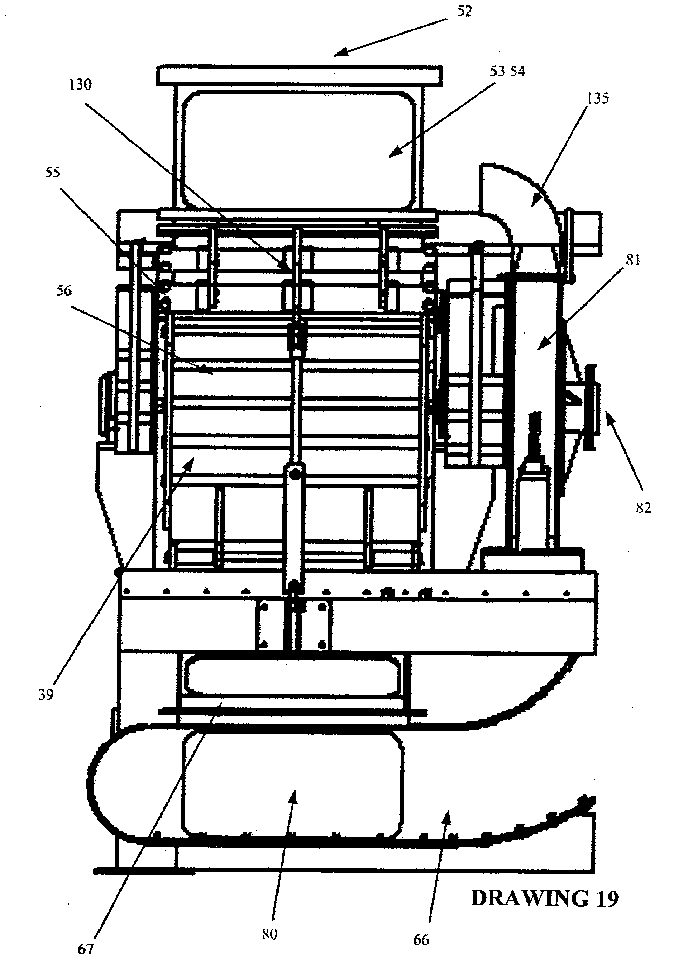

Drawing 19 presents the Low Frequency Pulsator (39) within the metallic construction it is housed in (55), the exit of the pulsator to the second chain conveyor (66) and the first centrifugal pump (81), with its entrance (82) and its exit (135).
Drawing 20 presents the second chain conveyor (66) leading the product to the High Frequency Pulsator (68) and the second centrifugal pump (96), with its entrance (97) and exit (100).

### DETAILED DESCRIPTION

A not restrictive application of the method and the system that applies it is described below with reference to the drawings.

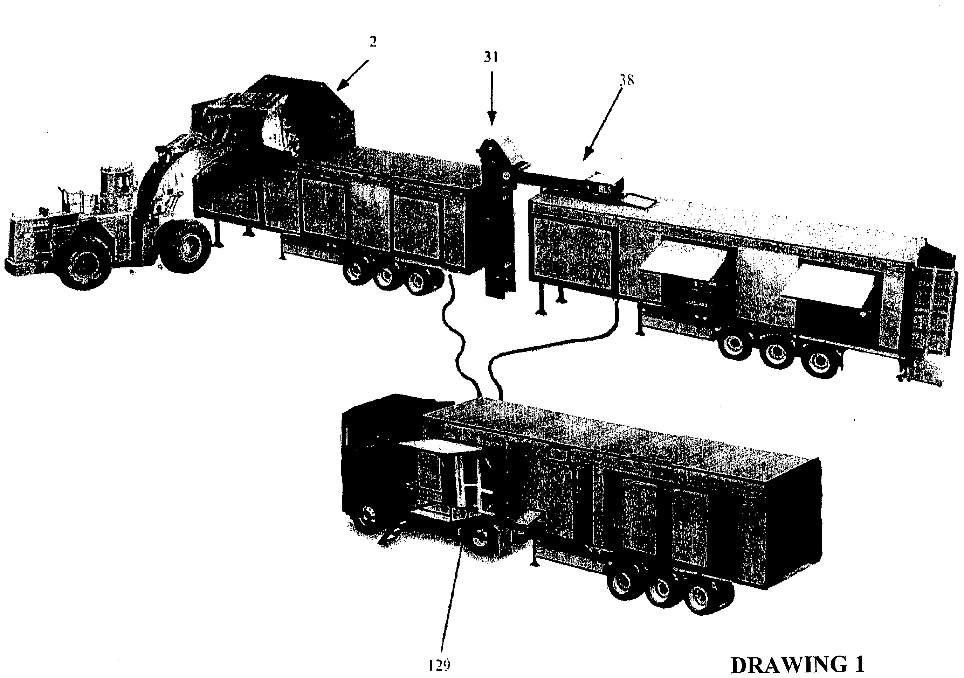

1st Step: Entrance of the wastes in the system. As shown in drawing 1, the Municipal Solid Waste is guided initially in the system, through a reception funnel (1). In the reception funnel (1) the waste is thrown either with the elevator or without, but also in an other way, e.g. with quick and great feeding capacity loaders or with funnels situated in the highest point, from where the waste falls directly to the reception funnel (1). The feeding of the waste to the funnel can be done directly with a garbage truck, or there can be more than one waste reception funnel (1).

In the system that applies the present method, the reception funnel (1) can be equipped with proper windproof sheets (2) (flaps) to avoid the scattering of the light objects during the unloading of the garbage truck in the surrounding area that might not be put in garbage bags. The windproof sheets (2) are made of resistant material like metal, plastic, etc., and constitute also a cover - loading gate to cover the free surface of the reception funnel (1) immediately after each loading procedure. The opening of the windproof sheets (2) (loading gate) can be done with a mechanical, electrical, hydraulic, pneumatic way, or even manually.

Additionally, the reception funnel (1) has at least one visiting manhole (3) (dr. 3) in case of need and maintenance.

The reception funnel (1) has a sufficient capacity for the system to be in continuous operation without dead periods of feeding and is equipped with electronic arrays, such as photocells (4) for material level control to avoid overfeeding.

Its shape is such that the cross section is square or rectangular; the dimensions of that cross section are decreasing from top to bottom in order to help the sliding and falling of the waste down and its conduit to the interior of the system.

In the reception funnel (1) there can be measurement and data recording arrays like the weight and the volume of the incoming waste, and they send the data in real time to the Control and Handling Unit where they are recorded. Thus, through the Control and Handling Unit a control is effectuated and the on-line observance of the whole procedure, with the possibility of intervention, from the initial reception of the waste until the completion of its treatment.

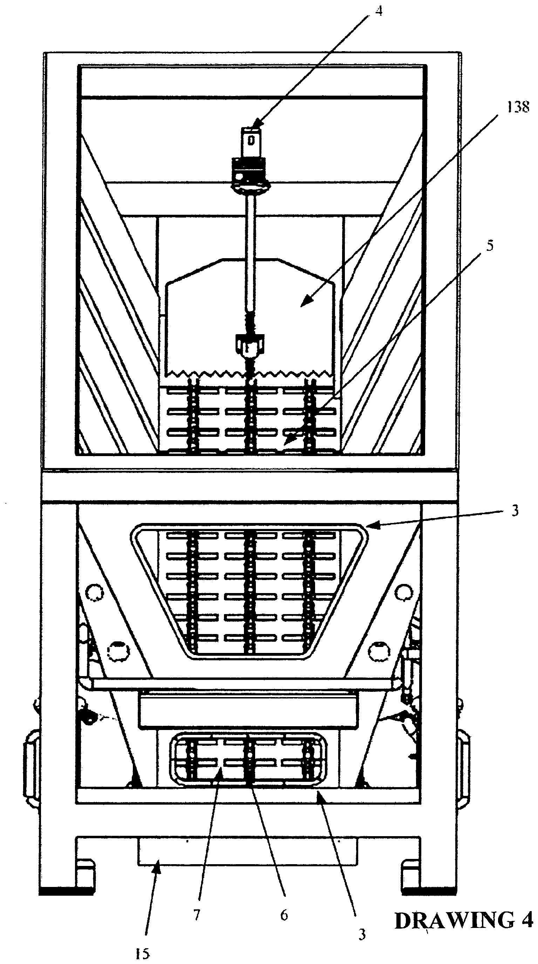

The inclined side parts of each reception funnel (1) end to their lower part at least to one first chain conveyor (5) (drawing 4). The first chain conveyor (5) constitutes the lower part of the reception funnel (1). The design and the reception funnel (1) dimensions are adequate to facilitate the smooth flow of waste to the first chain conveyor (5) and are sealed to avoid the small waste and odors escaping except the liquid drainage, to the drainage tank (15) described below. The reception funnel (1) accomplishes the gradual and smooth flow of waste to the first chain conveyor (5), which is of continuous operation.

In the present method, the first chain conveyor (5) (dr. 2, 4 and 5) begins from the reception funnel (1) and extends to the Homogenization Unit (28) and the entrance of the vertical pad elevator (31) or the horizontal conveyor (136) that intervenes and transports the product to the vertical pad elevator (31) and the following treatment stages. The transportation of waste to the following treatment stages can be achieved with the use of a conveyor belt.

In the present method and in the system applying it, the first chain conveyor (5) is of metallic resistant construction and of variable speed. However, it is equipped with flow control in order to avoid the Municipal Solid Waste congestion in the special opening unit (19). The flow control is effectuated with the known ways of today, such as with electronic array for the measurement of the electric current in the motor that rotates the special opening unit (19). As soon as the array detects the sudden increase of the electric current, it gives the order to the motion control array of the first chain conveyor (5) either to move slower or to stop all together.

In the case that a bulky object is led to the special opening unit (19) then the motion speed of the first chain conveyor (5) is modulated automatically.

Furthermore, the reception funnel (1) has a waste block system (138). The waste block system (138) (drawing 4) is a metallic surface which slides down to the level of the first chain conveyor (5), when it is considered necessary and it bars the flow of waste. The metallic surface can be regulated to a certain height from the first chain conveyor (5) and not to be moved again from that point except in special cases (e.g. if a bulky material falls in the reception funnel (1)).

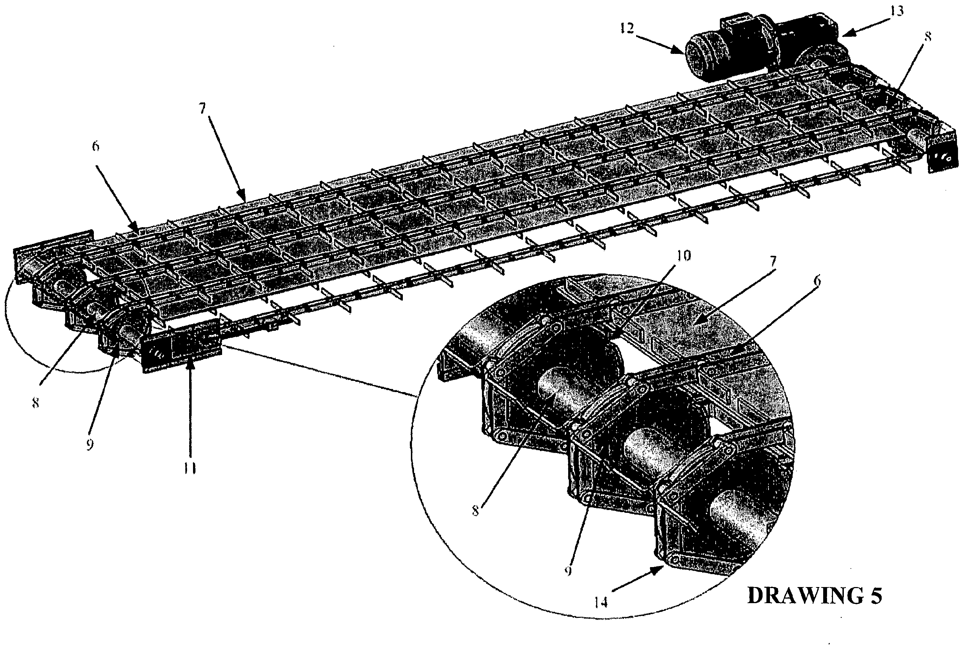

In the mechanical system that applies the present method, the first chain conveyor (5) (drawing 5) comprises at least one chain (6), which drives the waste to the following treatment stages, the sliding surface (7) of the chain (6), which constitutes the lower part of the system and two cylindrical axes (8) that bear vertically and concentrically proper serrated discs (9). These cylindrical axes (8) are situated in both ends of the sliding surface (7) and give motion to the chain (6). The serrated discs (9) of each cylindrical axis (8) are numerically equal to the number of chains (6) with the proper diameter. In their perimeter the discs (9) have recesses (10) of adequate size inside which every chain (6) rests, one on each serrated disc (9). The first cylindrical axis (8) situated in the beginning of the first chain conveyor (5), from the side of the reception funnel (1), is situated inside guides (11) which permit it to move horizontally as to the level of motion of the first chain conveyor (5). The movement of the cylindrical axis (8) applies and removes tension on the chain (6) of the first chain conveyor (5) depending on the direction, relative to the motion of the chain (6), that the user will select to move it. The second cylindrical axis (8) is axially connected with the electric motor (12) through the proper reducer (13) and transfers regulated movement to the chain (6) of the first chain conveyor (5). Other ways of motion transfer are possible, like for example a belt and pulley array or chain and gears.

Each chain (6) of the first chain conveyor (5) comprises either articulate metallic sheets of proper width or an integrated synthetic auger (14) with horizontal plates that facilitate the promotion and measuring of the waste.

Under the first chain conveyor (5) and along the reception funnel (1), the system comprises a drainage tank (15) (drawing 4) for the drainage collection where most of the volume of liquids is collected originating from the Municipal Solid Waste during their insertion on the reception funnel (1). The sliding surface (7) of the first chain conveyor (5) constitutes the upper part of the drainage tank (15) for the drainage collection. A way for the liquid drainage to be led within the drainage tank (15) is along the reception funnel (1) the sliding surface (7) to be made of perforated sheet metal in order to allow liquid drainage to flow inside the drainage tank (15). At the same time, it will not permit waste bigger than the cross-section of its holes to enter into the interior of the drainage tank (15). In the case the sliding surface (7) is made of compact sheet metal, the collection of the drainage can be achieved by creating on the sliding surface holes adequate in number and dimension through which the liquid drainage will flow to the interior of the drainage tank (15). Alternatively and in case it is preferable to use a compact sheet metal for the construction of the sliding surface (7) then in order to guide the liquids inside the drainage tank (15) the first chain conveyor (5) must have a slight gradient. The gradient must be opposite to the movement of the Municipal Solid Waste so as, while they are moving, the liquid drainage will be guided to the back to the start of the first chain conveyor (5). There will be an adequate array (e.g. opening in the sliding surface) that will lead the drainage inside the drainage tank (15). In any case, however, the existence of a small gradient is desirable on the first chain conveyor (5) for the liquid drainage to be guided in the drainage tank (15) along with other liquids resulting in the following stages of the treatment (in the Special Opening Unit (19) and the Special Homogenization Unit (28)), given the fact that in the waste there is usually a great quantity of liquids.

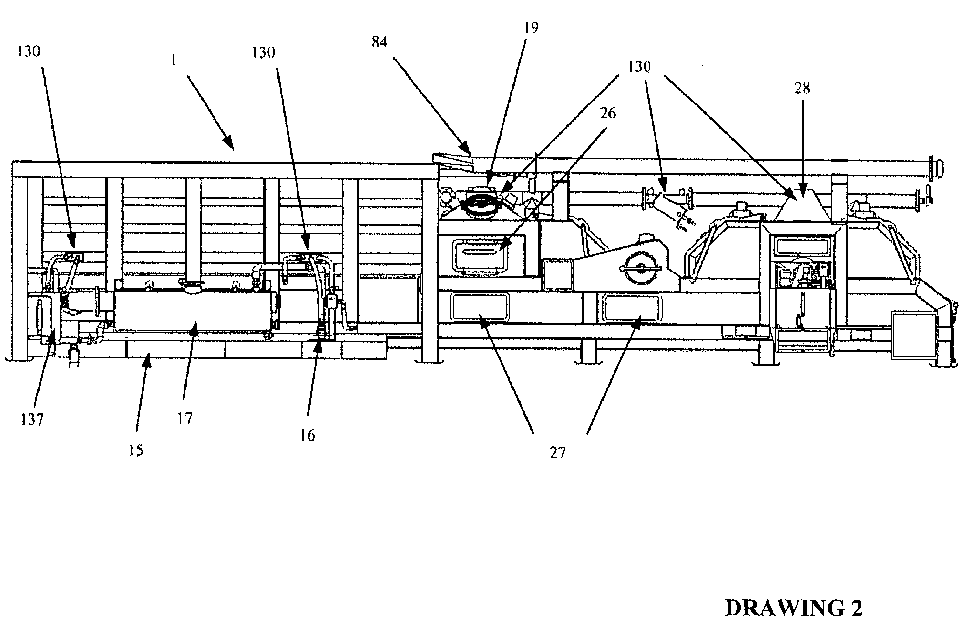

In the drainage tank (15) (drawings 2, 3 and 4) there is at least one special pump (16) for thick liquids, which guides thick liquids in the liquid drainage tank (15), to at least one separation system (17) existing outside the reception funnel (1).

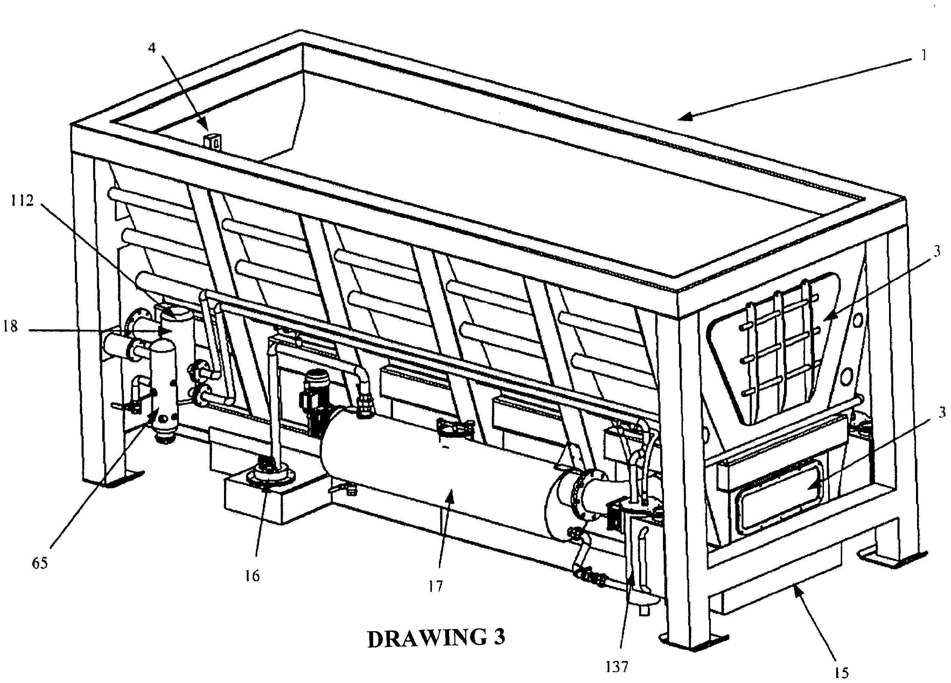

The separation system (17) (drawing 3) guides any solids inside the Municipal Solid Waste reception funnel (1) or, alternatively, to an exterior collector while the liquids are guided through pumps (137) to at least one liquid drainage storage tank (18). The liquid drainage storage tank (18) includes in its interior a filter of active carbon (112) for the cleaning of the liquids. Then and through at least one additional filter (65), the spraying nozzles (113) are spraying the liquids, if and when necessary, through adequate tubing, in the reception funnel (1), the Low Frequency Pulsator (39), the High Frequency Pulsator (68) and the Stabilization Chamber (83). The reuse of the liquids through spraying is useful:
(a) to decrease the possibility of a fire
(b) to increase the material conductivity
(c) to increase the humidity in order to achieve the final decrease of volume of the material and the production of pellets at the end of the procedure.

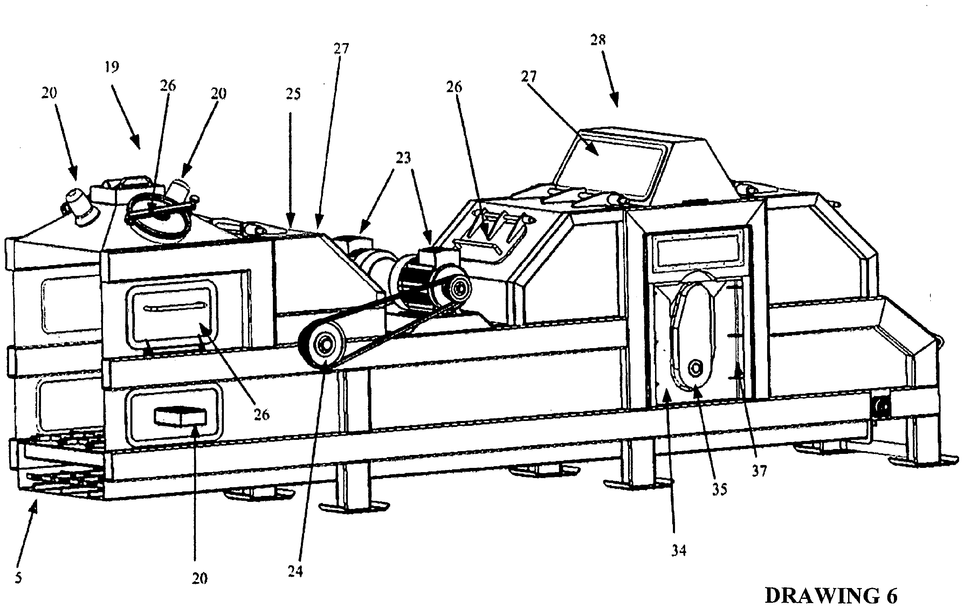

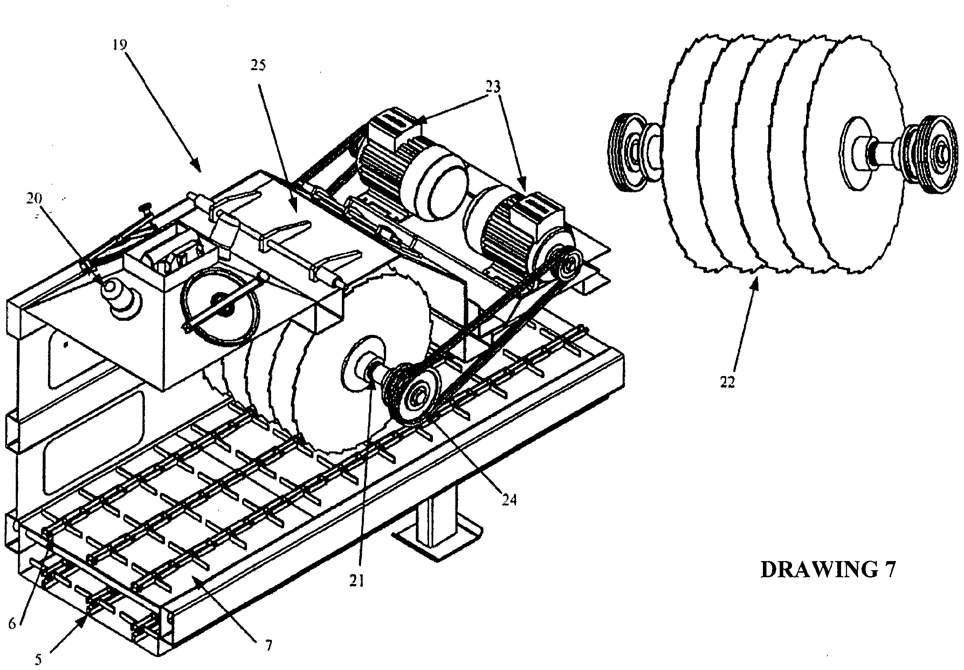

As the application of the method begins, the first chain conveyor (5) leads the waste, the majority of which is packaged in garbage bags or are loose outside them, to the Special Opening Unit (19) (drawings 6 and 7), where the opening of the Municipal Solid Waste packaging is achieved (bags, cartons etc.) and the cutting of bulky waste with a mechanical cutting method.

At a proper point, between the reception funnel (1) and the Special Opening Unit (19), there is a visual control system (20). The visual control system (20) controls the waste before it is guided to the Special Opening Unit (19). It is a visual control system (20) programmed according to the size and shape parameter or/and visual control system of movement/alive or dead organism, which stops the operation of the machine, if there is a reason for it (e.g. a structural material of large dimensions, a material that is moving) and transfers data to the Control and Handling Unit (129), (dr. 1). The visual control system (20) can also include an X-RAY system. The visual control can also be effectuated by the personnel situated at the Control and Handling Unit (129) through screens. The control is done without the procedure being stopped. The visual control system (20) stops the operation of the machine if there is reason for it. The operation of the machine can also be stopped by the user of the Control and Handling Unit (129), if he thinks it is necessary.

### 2^{nd} Step: Treatment of opening and cutting by using the special opening unit (19).

As shown in Drawing 7, the Special Opening Unit (19) possesses a rotating axis (21) with circular serrated cutting discs (22) (knives - cutters). The cutting discs (22), can be made of stainless steel, while the parts that bear increased mechanical loads (like for example the rotating axis (21) are made of materials of adequate resistance. The waste, inside or outside of bags, is transported on the first chain conveyor (5) and on the rotating knives of the Special Opening Unit (19), which cut the waste packaging and their content. The rotating axis (21) is moving in a position horizontal and parallel to the level of the first chain conveyor (5). The vertical distance between the first chain conveyor (5) and the cutting discs (22) is such that bulky objects cannot pass without being cut, with the possibility this vertical distance to be fluctuated by the user. In the case that we desire the recycling of whole packages (e.g. plastic or glass bottles) the user can change the whole system of axis-knives, and place one that has a bigger distance between the knives, so for the objects to "pass" without being cut.

The cutting of the waste takes place without the movement of the first chain conveyor (5) to be necessary to stop at any point. Depending on the waste volume existing in the Special Opening Unit (19) the speed of movement of the first chain conveyor (5) can also be regulated, according to the known today's state of the art of electronic array of electric current measurement in the motor that rotates the special opening unit (19). As soon as the array detects a sudden increase of the electric current value, gives an order to the array of movement control of the first chain conveyor (5) to move slower or even to stop completely.

The Special Opening Unit (19) receives motion from at least one motor (23) through a pulley and belt array (24). The rotation speed of the cutting discs (22) can also be regulated.

The existence or not of serrations on the cutting discs (22), the number of cutting discs (22) in the Special Opening Unit (19) as well as the material they are made of, and also the distance between them are not restrictive elements for the application of the present invention and can vary according to the choice of the user.

The opening of the waste packages is done by the Special Opening Unit (19) which possesses all the necessary protective measures for the avoidance of accidents. It has protection lids (25) and access hatches (26) (drawing 6) to facilitate the maintenance and repairs works. It also has see-through windows (27) to facilitate the visual control of the procedure if necessary.

### 3^{rd} Step: Homogenization treatment by using the special homogenization unit (28).

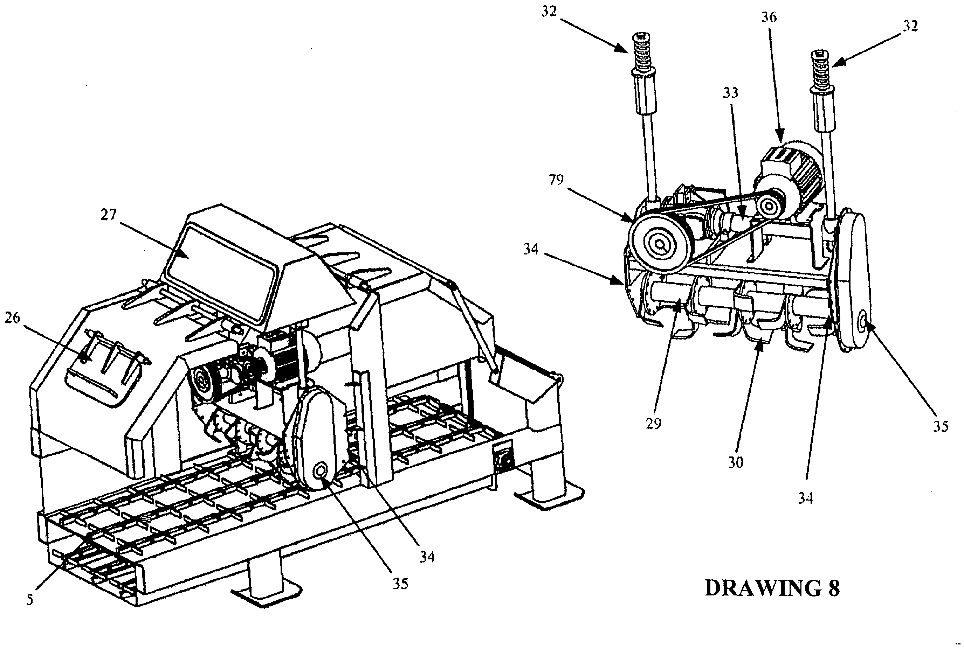

Then, for the application of the present method, the initially cut waste is transferred by the first chain conveyor (5) to the Special Homogenization Unit (28) (drawings 6 and 8).

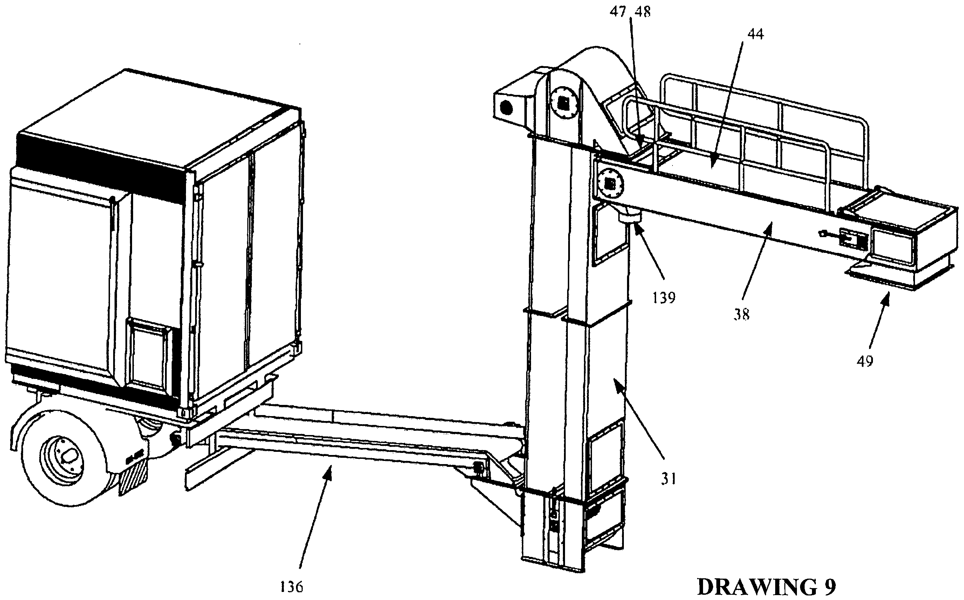

As seen in Drawing 8, the system that applies the method possesses as a component the Special Homogenization Unit (28) which includes a rotating axis (29) with multiple metal plates (30) / knives. The rotating axis (29) is horizontal and parallel to the level of the first chain conveyor (5). On the horizontal rotating axis (29) multiple metal plates (30) are placed which constitute the homogenization means. There, the initially shredded waste, is additionally cut into pieces of smaller dimension and then they are led again by the first chain conveyor (5) to the vertical pad elevator (31) (drawing 9) and from there to the next stage, with or without the intervention of a horizontal conveyor (136) (depending on whether the selection of the recyclable materials is desired at this stage or not).

The rotating axis (29) of the Special Homogenization Unit (28) is consolidated on the system with a metallic frame. The frame comprises a horizontal supporting axis (33) and two lateral supports (34). The horizontal supporting axis (33) is placed at such distance above the rotating axis (29), that will not hinder its rotation and by extension the homogenization of the product.

The two axes (29, 33) are parallel to each other. The two lateral supports (34) are parallel to each other and situated on either side of the horizontal supporting axis (33), vertically to it. The rotating axis (29) is supported with a bearing array (35) by the two lateral supports (34). The rotating axis (29) rotates by an electric motor (36) and arrays of belts - pulleys or electric reducer (79). The horizontal supporting axis (33) on its upper part is connected with the rest of the system with at least two arrays with respective coil springs (32). The two lateral supports (34) are situated within vertical guides (37)

(drawing 6). The arrays with the coil springs (32) (drawing 8) and the vertical guides (37) to the lateral supports (34) permit the system of Special Homogenization Unit (28), to move vertically if needed, and particularly in case that some bulky object passes by the Special Opening Unit (19) without being fully cut, the Special Homogenization Unit (28) has the capability to move vertically and avoid damage or/and jamming in the system while in parallel it will cut the object into smaller pieces.

The product coming after the Special Homogenization Unit (28) is cut in pieces of similar size and continues its course always guided by the first chain conveyor (5) to the following stages.

A system of visual control can be placed even before the Special Homogenization Unit (28), for the control of the incoming waste. The Special Homogenization Unit (28) possesses itself respective access gates (26) and see-through windows (27) (drawing 6). The product comes out of the Special Homogenization Unit (28) and in case a horizontal conveyor (136) does not exist (drawing 9), it is guided to the entrance of the vertical pad elevator (31).

If a horizontal conveyor (136) is used then the waste from the Special Homogenization Unit (28) is first led to the horizontal conveyor (136) and then, by it, to the vertical pad elevator (31). As mentioned above, the horizontal conveyor (136) is used in the case that the selection of recycled materials is desired at this stage. In the case that the selection of recyclable materials is not desired at this stage, the horizontal conveyor (136) is not used and the product is guided directly to the vertical pad elevator (31) and from there to the horizontal measuring feeder (38).

### 4^{th} Step: First separation of the ferrous metallic elements with magnetic elements.

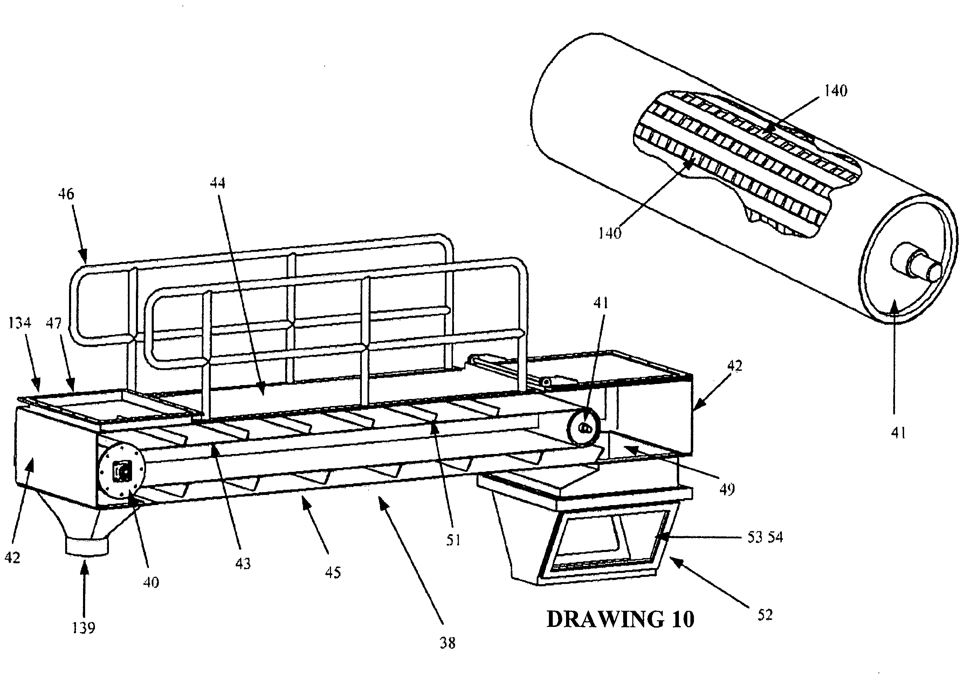

As shown in Drawing 9 the vertical pad elevator (31) lifts and guides the shredded product to the horizontal measuring feeder (38) (dr. 2, 9 & 10). The horizontal measuring feeder (38) is a conveyor belt which feeds the product to the first Low Frequency Pulsator (39) (dr. 17), controlling the feeding flow in order to avoid jamming in the feeding of the product to the Low Frequency Pulsator (39). In particular, as depicted in drawings 9 & 10, the horizontal measuring feeder (38) feeding the shredded product to the first Low Frequency Pulsator (39) comprises two rotating axes (40), (41), from the metallic frame (42) and from the conveyor belt (43). The horizontal measuring feeder (38) is of the closed type as on its upper part it has a lid (44) and adequate bottom (45). The one rotating axis (40) is rotating by an electric motor and reducer array (134). On the lid (44) of the horizontal measuring feeder (38) there are safety rails (46), to provide safety in case someone has to walk on the horizontal measuring feeder (38) to execute any work or control. With the rotation of the rotating axis (40) the conveyor belt (43) of the product is moving. The second rotating axis (41) is placed in a way to freely rotate. The entrance (47) (dr. 9 and 10) of the horizontal measuring feeder (38) is connected to the exit (48) of the vertical pad elevator (31) from where the shredded waste is coming, while the exit (49) of the horizontal measuring feeder (38) is connected with the entry (50) of the Low Frequency Pulsator (39).

The conveyor belt (43) of the product possesses pads (51) vertically or in any other angle to it. The pads (51) drive the product towards the exit (49) of the horizontal measuring feeder (38) and the entry (50) of the Low Frequency Pulsator (39). The pads (51) have an equal length to the width of the conveyor belt (43). The height of the pads (51) is such that there is no space between the bottom (45) and the edge of the pads (51).

The rotating axis (41) situated over the exit (49) of the horizontal measuring feeder (38) has many magnetic elements (140) (dr. 10) which magnetize the ferrous metals existing within the product. By the magnetizing force exercised by the magnetic elements (140) to the ferrous metals of the product while it passes over the rotating axis (41), the ferrous metals remain on the conveyor belt (43) and do not fall as soon as they are over the exit (49) of the horizontal measuring feeder (38) while the remaining materials do fall. However, as soon as the specific point of the conveyor belt (43) where the magnetized metals are, surrounds the rotating axis (41) with the magnetic elements (140) and passes it, the metals are not attracted anymore by the magnetic elements (140) and are free to the bottom (45) of the horizontal measuring feeder (38) separated from the other materials that fell from the exit (49) and were guided to the Low Frequency Pulsator (39). There, they are driven by the pads (51) of the conveyor belt (43) with the adequate height, and pushed by the pads (51), they move to the lower side of the conveyor belt (43) in a direction opposite to the movement direction of the product on the upper side of the conveyor belt (43) (drawing 10). In this way, the selected ferrous metals are guided and exit the horizontal measuring feeder (38) through a proper exit (139) (drawing 10) situated on its lower side (45). From there, they are guided to any adequate collection array, constituting this product clean ferrous metal ready for recycling. This step of the method is optional. If the selection of the ferrous metals is not desired at this stage of the method, the rotating axis (41) does not bear magnetic elements (140) and the Horizontal Measuring Feeder (38) does not have on its lower part (45) the second exit (139).

### 5^{th} Step: Treatment of first pulverization by using a Low Frequency Pulsator (39)

As shown in Drawings (10 and 17), the Low Frequency Pulsator (39), is fed with the product from the horizontal measuring feeder (38) through a feeding funnel (52). The feeding funnel (52) can be rectangular, the cross section of which is decreasing at the bottom. The shape of the feeding funnel (52) is such as not to hinder in any case the smooth flow of the product to the Low Frequency Pulsator (39). The feeding funnel (52) has access gates (53) and supervision windows (54).

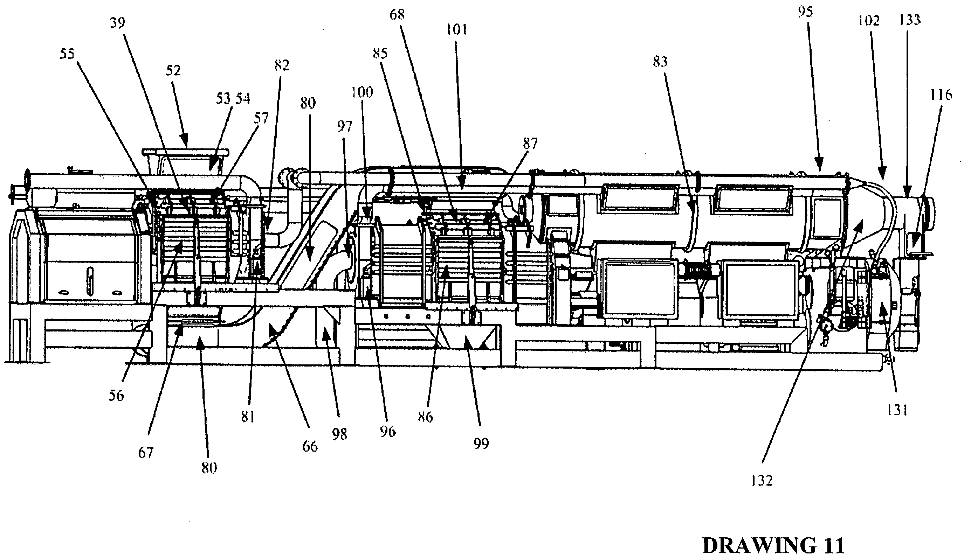

The Low Frequency Pulsator (39) (dr. 17) is placed within a robust metallic construction (55). The metallic construction (55) that includes the Low Frequency Pulsator (39) has an entrance opening (50) on its upper part, where the exit (52) of the feeding funnel is connected. It also has an exit opening (67) on the lower part from where the product exits and goes to the following stages of the treatment after having passed from the Low Frequency Pulsator (39). The metallic construction (55) that includes the Low Frequency Pulsator (39) has at its sides the proper hatches (56) and access windows (57) (drawing 11) to facilitate the work of the operators in case access/visit is needed to the Low Frequency Pulsator (39) for reasons of maintenance, repair or/and component replacement of the Low Frequency Pulsator (39).

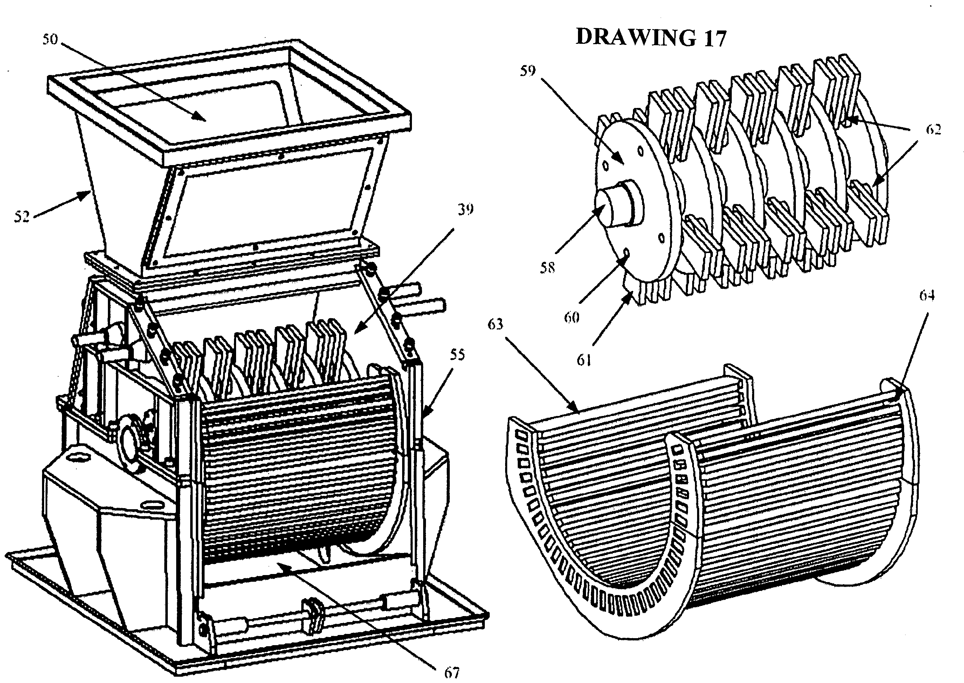

As shown in drawing 17, the Low Frequency Pulsator (39) comprises a rotating axis (58) that comes vertically from at least two metallic supporting discs (59) with an adequate distance between them. On their perimeter and at a proper distance from their center, the supporting discs (59) bear cylindrical axes (60), vertical to their surface, on which pulse elements (61) are placed. The supporting discs (59) are parallel to each other. The cylindrical axes (60) bearing and supporting the pulse elements (61) are vertical to the supporting discs (59) and parallel to the rotating axis (58). The pulse elements (61) are placed on the cylindrical axes (60) in the space created between the supporting discs (59). The distance between the pulse elements (61) is determined by a perforated rotating axis (62) of adequate length and of cylindrical shape. The frequency of the pulses applied on the product that comes in the Low Frequency Pulsator (39) and therefore the granulometry of the pulverized material also, depend on the number of the cylindrical axes (60) bearing the pulse elements (61) but also on the number of the pulse elements (61) that each cylindrical axis (60) bears. The application of pulses on the product results to further shredding, the breaking of its mass and volume and the partial destruction of microorganisms.

Under the rotating axis (58), on the lower part of the metallic construction (55) there is a metallic array of horizontal control elements (63) controlling the volume, which have a specific width and are situated in determined distances between them creating openings (64). The volume of the product must be shredded to such a degree so as to be able to enter into the openings (64) of the horizontal control elements (63). As soon as the product is of desired granulometry it passes through the openings (64) of the horizontal control elements (63) and exits from the Low Frequency Pulsator (39) towards the following stages of treatment.

The user has the option to modulate the openings (64) of the horizontal control elements (63), by replacing the array with another with smaller or bigger openings. The choice of the adequate granulometry depends on the way of exploitation the user has in mind for the final product. As a general maximum application of the method is suggested, if the user, for example, desires the production of fuel, then the product must be of low granulometry, while if the user desires the production of soil improving material, then the product must be of larger granulometry, etc.

The horizontal control elements (63) can have rectangular shape with edges from the internal side, where the edges contribute to the shredding of the material, while as internal side is considered to be the side that faces the interior of the Low Frequency Pulsator (39).

When the product passes from the horizontal control elements (63) of the Low Frequency Pulsator (39) it is guided to its lower part to the exit (67) of the array and falls free by gravity to the second chain conveyor (66) to lead it towards the High Frequency Pulsator (68) which is the next stage of treatment.

The exit (67) of the array is of a conical shape with its diameter shortening from top to bottom.

The product that comes out from the Low Frequency Pulsator (39) is guided by the second chain conveyor (66) to the High Frequency Pulsator (68) which is the next stage in the treatment.

### 6^{th} Step: Further pulverization treatment by using the high frequency pulsator (68).

The transfer of the product from the Low Frequency Pulsator (39) to the High Frequency Pulsator (68) takes place by the second chain conveyor (66), through which a violent artificial flow of air is created because of the operation of the second centrifugal pump (96) for which there is reference below (dr. 11). The use of the chain conveyor is not necessary for the application of the method, as the transfer of the product could be accomplished by other means.

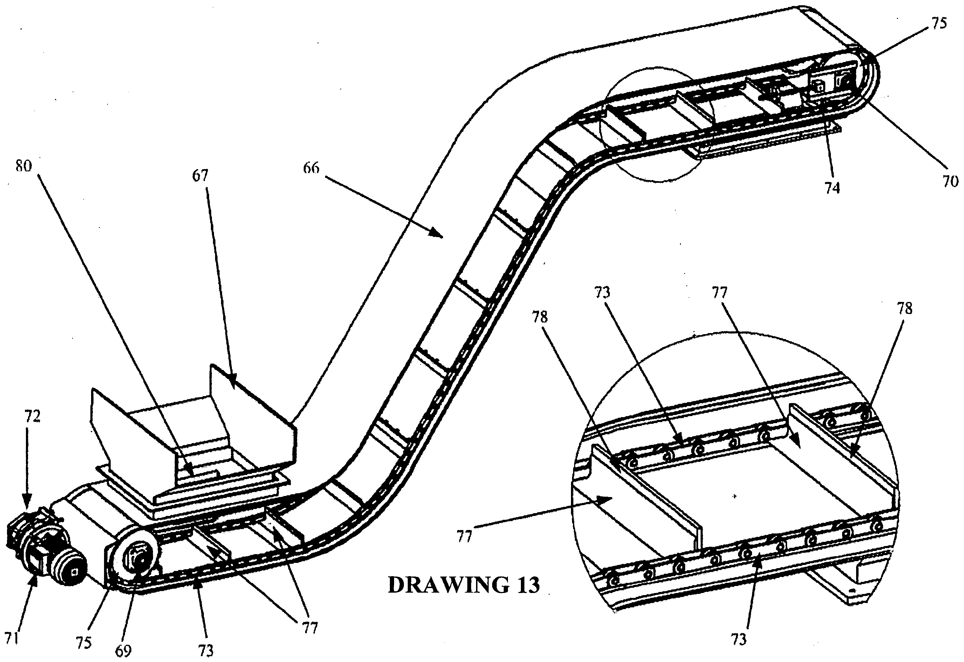

As shown in drawings 11 and 13, the second chain conveyor (66) can have a gradient in order to transport the product from the lower part of the low frequency pulsator (39) to the upper part of the high frequency pulsator (68) if between them there is a height difference. It is of a closed type so as not to loosen dust and smells to the atmosphere. The inclined second chain conveyor (66) has a rotating axis (69), (70) at both ends.

The rotating axis (69) situated under the Low Frequency Pulsator (39) is connected to an electric motor (71) through an adequate reducer (72) and transfers motion to the chain (73) of the second chain conveyor (66). Other ways of motion transfer are known and can be used without affecting the application way of the present method. The rotating axis (70) situated over the High Frequency Pulsator (68) is placed within guides (74) which permit its horizontal movement regarding the level of movement of the second chain conveyor (66). The movement of the rotating axis (70) applies and removes tension to the chain (73) of the second chain conveyor (66), depending on the direction, relative to the movement of the chain (73), chosen by the operator.

If the operator chooses to move the axis to the right, relative to the movement of the chain (clockwise), then he removes tension from the chain (he releases it), while if he makes the opposite then the result will be that the chain will be tighter.

Each rotating axis (69), (70) includes at each end one serrated disc (75) of proper width. The two serrated discs (75) of each rotating axis (69), (70) are vertical as to the rotating axis (69), (70) and therefore parallel to each other. Each serrated disc (75) has on its surface recesses of proper dimensions on which each chain (73) rests. The serrated discs (75) of each axis are equal in number to the chains (73) of the second chain conveyor (66), and this way, if for example the chains are three, the discs on each axis are also three. In this case two chains (73) are used. The rotation of the rotating axes (69), (70) results to the movement of the chains (73) thanks to the serrated discs (75). At regular intervals and at a proper distance between them, metallic plates (77) are placed vertical as to the chains (73). Generally, it is suggested, for the best application of the method, the metallic plates (77) to be placed at a distance from each other that varies from around 40cm to 80 cm. Each metallic plate (77) is connected on either side with the chains (73) at a vertical position to them. On each metallic plate (77) and to the interior of the second chain conveyor (66) a stripe of material (78), along the metallic plate (77) is placed. The stripe of material (78) is the element which drags and transfers with the movement of the chains (73) the product to the next stage. It has length equal to the length of the metallic plate (77). This stripe of material (78) can be made of various materials such as plastic, metal etc. The material comes into the second chain conveyor (66) from the upper side of its lower part, which has an opening of the same dimensions with the exit of the array where the Low Frequency Pulsator (39) (drawing 13) is included. The product passes through the gap created by the metallic plates (77) situated among the chains (73) in the interior of the second chain conveyor (66) where it is dragged by the stripes of material (78) and guided to the following stages of the treatment. The movement of the second chain conveyor (66) is transferred through a motor array (71) and a reducer (72) and the speed can be regulated depending on the volume of the product to be processed in the interior of the High Frequency Pulsator (68). Thus, if the High Frequency Pulsator (68) has a lot of material to process, then the speed of the chain conveyor is regulated to be slow, while if it has less material, then the speed is regulated to be higher. The second chain conveyor (66) at adequate point has access hatches - windows (80) for the intervention of the personnel in case of emergency and the observance of the procedure.

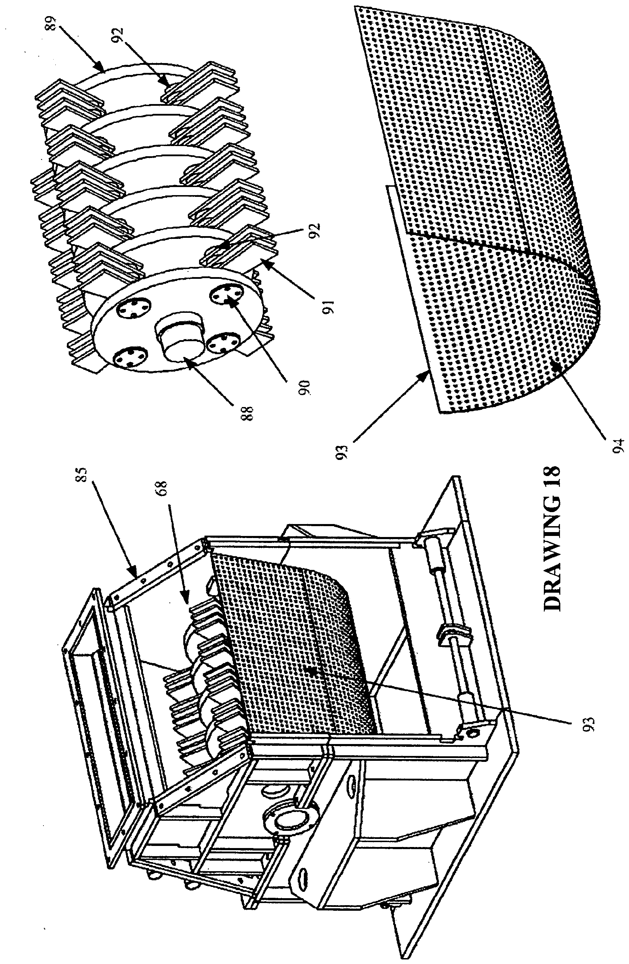

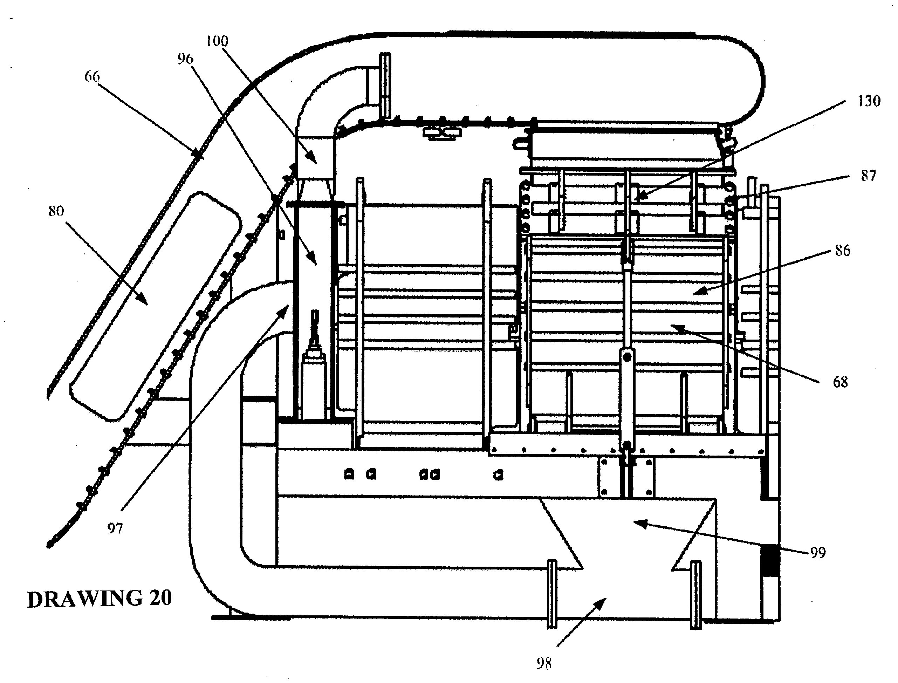

As shown in drawing 18, the High Frequency Pulsator (68) is also placed inside a robust metallic construction (85). The metallic construction (85) that includes in its interior the High Frequency Pulsator (68) has an opening on its upper part, where the exit of the second chain conveyor (66) is connected that transfers the product (drawing 20). It also has an opening on the lower part from where the product exits towards the next stages of the treatment. The metallic construction (85) that includes the High Frequency Pulsator (68) has on its sides adequate hatches (86) and access windows (87) (drawing 11) to facilitate the work of the operators in chase where access is necessary for maintenance, repair, or replacement of components of the High Frequency Pulsator (68).

The High Frequency Pulsator (68) (drawing 18) develops pulses of higher frequency than the first Pulsator (39). It comprises a rotating axis (88), coming vertically from the center of at least two metallic supporting discs (89) with some distance between them. In their perimeter and at an adequate distance from their center the supporting discs (89) bear cylindrical axes (90) vertical to their surface, where the pulse elements (91) are placed and supported. The number of the pulse elements (91) on the High Frequency Pulsator (68) is equal or higher than the number of the elements (60) of the Low Frequency Pulsator (39). The supporting discs (89) are parallel to each other and vertical to the rotating axis (88). The cylindrical axes (90) bearing the pulse elements (91) are vertical to the supporting discs (89) and parallel to the rotating axis (88). The pulse elements (91) are placed on the cylindrical axes (90) at the space created between the support discs (89). The number of the pulse elements (91) on the High Frequency Pulsator (68) is equal or higher than the number of the pulse elements (61) of the Low Frequency Pulsator (39). The distance between the pulse elements (91) is determined by a perforated cylindrical axis of adequate length (92). The number of the cylindrical axes (90) bearing the pulse elements (91) but also the number of the pulse elements (91) that each cylindrical axis (90) bears determine the frequency of the pulses applied on the product incoming to the interior of the High Frequency Pulsator (68).
In any case and since the High Frequency Pulsator (68) possesses a greater number of cylindrical axes (90) as much as of pulse elements (91) than the Low Frequency Pulsator (39), it develops pulses of higher frequency, even in the case of equal or slower rotation.

If, however, the High Frequency Pulsator (68) possesses an equal number of cylindrical axes (90) and pulse elements (91) as to the Low Frequency Pulsator (39), then the High Frequency Pulsator (68) must rotate faster so as to develop pulses of higher frequency than the Low Frequency Pulsator (39), a desired fact for the application execution of the method.

The application of higher frequency pulses on the product results to the further shredding, breaking of mass and volume and the further destruction of microorganisms.

As similarly with the Low Frequency Pulsator (39), with the High Frequency Pulsator (68) before the exit of the pieces to the next stages at its lower there is a metallic array of volume control elements (93) (drawing 18), which has a specific width. The volume of the product must be broken to such a degree that it will be able to pass from the openings (94) of the array of the control elements (93). As soon as the product reaches the desired granulometry it passes through the openings (94) of the array of control elements (93) and exits from the High Frequency Pulsator (68) towards the next stages of the treatment. The openings (94) of array of control elements (93) of the High Frequency Pulsator (68) are of smaller dimension that the openings (64) of the horizontal control elements (63) of the Low Frequency Pulsator (39) (drawing 17). Through these openings (94) comes the product towards the next stages of the treatment.
The openings (94) may have various shapes and dimensions, suffice the product to pass easily. The dimension of the openings is selected depending on the desired granulometry of the outgoing product, according to what is mentioned above about the granulometry.

When the product passes from the array of control elements (93) of the High Frequency Pulsator (68) it is guided from its lower part, through a system of air transfer, towards the exit of the array and the next stages of the treatment.
The outgoing product from the High Frequency Pulsator (68) is now of fibrous form and free of microorganisms.

The system possesses a first centrifugal pump (81) (dr. 11) situated outside the High Frequency Pulsator (39).
The transfer of the product from the High Frequency Pulsator (68) to the Stabilization Chamber (83) is done by the air transfer system, which comprises the second centrifugal pump (96) (dr. 11) of the High Frequency Pulsator (68) and tubing that transfer the product and the air to the various stages of the treatment.

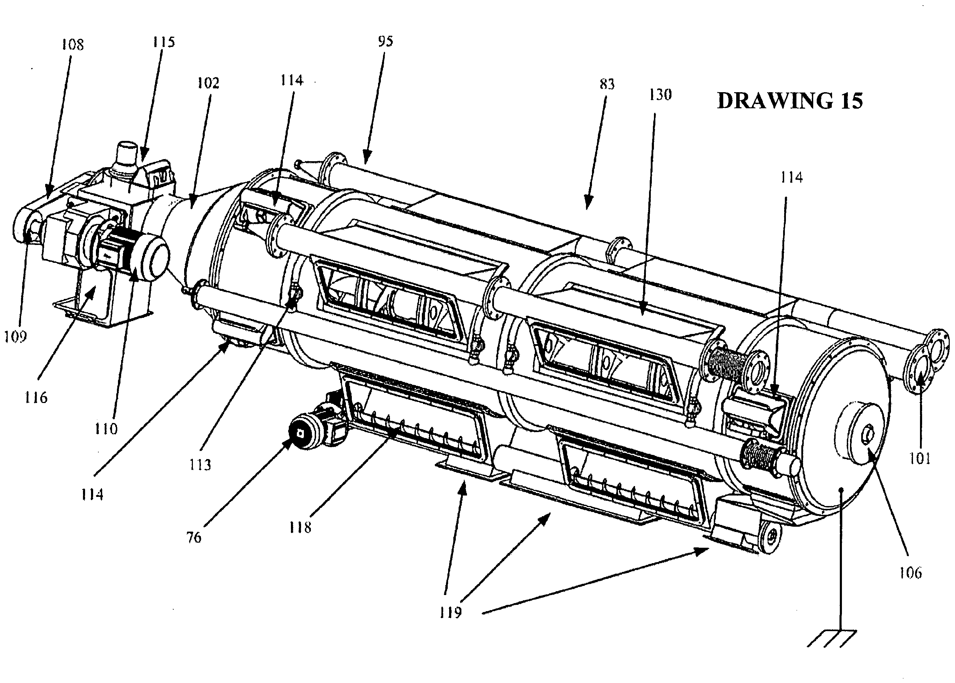

As seen in drawing 11 and in drawing 15, coaxially to the Low Frequency Pulsator (39) there is the first air centrifugal pump (81), of pressure and sub-pressure, which can be operated by a motor, through an axis and pulley array connected to each other with belts. The entrance (82) of the first air centrifugal pump (81) is connected with the Stabilization Chamber (83). From the Stabilization Chamber (83) the first air centrifugal pump (81) removes the excess air created in the interior of the Stabilization Chamber (83). At the same time with the excess air it removes the excess humidity from the product inside the Stabilization Chamber (83). The volume of the air enriched with the humidity removed from the product inside the Stabilization Chamber (83), is guided through a multiple filter array (84) (dr. 2) to the atmosphere. The special fine-grained filters from which the air passes throughout the atmosphere detain dust and particles floating in the air. They also remove the air from the humidity it carries.

Also, coaxially to the High Frequency Pulsator (68) (dr. 18 and 20), the second air centrifugal pump (96) (dr. 11 and 20) is connected of high pressure and sub-pressure which similarly to the first air centrifugal pump (81) is moved by a motor through an axis and a pulley array connected to each other with belts.

As shown in drawings 11 and 20, the entrance (97) of the second air centrifugal pump (96) is connected to the exit of the High Frequency Pulsator (68). The connection of the entrance (97) of the second air centrifugal pump (96) to the exit of the High Frequency Pulsator (68) is done through an adequate tube (98). The exit of the High Frequency Pulsator (68) ends to a conical funnel (99) whose shape and dimensions ensure the smooth flow of the product to the tube (98) resulting to its smooth movement to the centrifugal pump (96). The exit (100) of the second air centrifugal pump (96) is connected through tubing (101) with the rear part of the Stabilization Chamber (83). The tubing (101) leads the product from the exit (100) of the second air centrifugal pump (96) to the rear part of the Stabilization Chamber (83). The second air centrifugal pump (96) creates a strong air draught with a direction from the High Frequency Pulsator (68) to the Stabilization Chamber (83). This results to the violent transfer of the product from the High Frequency Pulsator (68) to the Stabilization Chamber (83).

As mentioned before, the first air centrifugal pump (81) situated outside the Low Frequency Pulsator (39), the second air centrifugal pump (96) connecting the High Frequency Pulsator (68) with the Stabilization Chamber (83) and the total of tubing transferring the product and the air to the various stages of the treatment constitute the air transfer system.
The second air centrifugal pump (96), which transfers the product from the High Frequency Pulsator (68) to the Stabilization Chamber (83), creates in the interior of the Stabilization Chamber (83) excess air. The excess air created by the second air centrifugal pump (96) inside the Stabilization Chamber (83) is removed from the first air centrifugal pump (81) which removes the air from the interior of the Stabilization Chamber (83).
The air draught created by the two air centrifugal pumps (81 & 96) of high pressure and sub-pressure, crosses the interior of the Stabilization Chamber (83) before it is guided to the special filter array (84) and then to the atmosphere. The product accumulated to the interior of the Stabilization Chamber (83) constitutes a natural filter which detains all the harmful organisms from the air draught that travel through the Stabilization Chamber (83). The Stabilization Chamber (83) constitutes a natural filter for the air mass of the air transfer of the system.
The air transfer of the fibrous product from the High Frequency Pulsator (68) to the Stabilization Chamber (83) causes the violent and quick dehumidification of the product along with the decrease in volume and mass. This happens because of the exposure of the product to the strong air draught during its transfer through the tubing and also during its stay inside the Stabilization Chamber (83) as the air transfer system creates and air draught inside the Stabilization Chamber (83) also.

### 7^{th} Step: Exposure to an electric field.

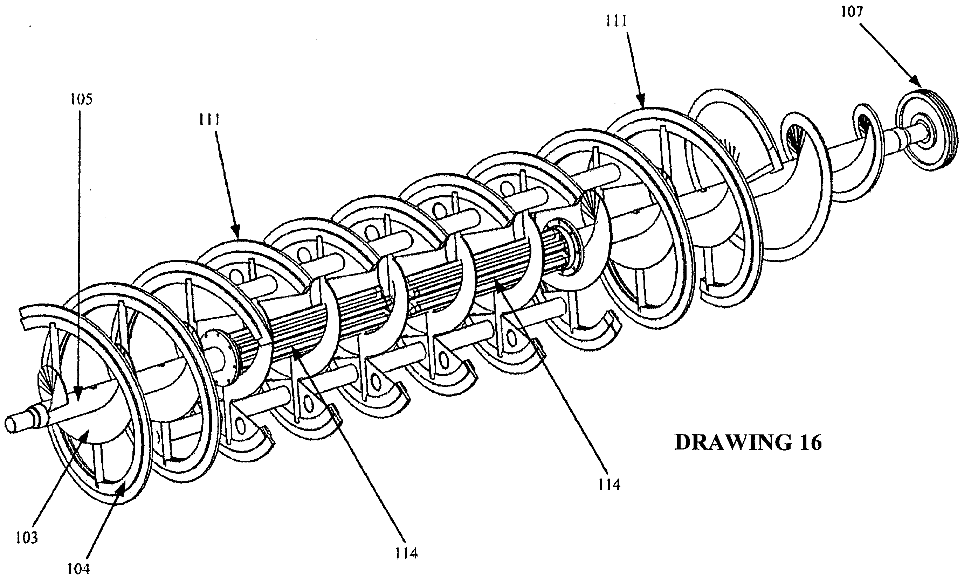

The product treated by the High Frequency Pulsator (68) comes into the Stabilization Camber (83) to the rear of the Chamber (83). As rear part (95) of the Stabilization Chamber (83) is considered the most remote part from the High Frequency Pulsator (68). The product coming out of the High Frequency Pulsator (68) is in the form of fibers and includes elements of various diameters. These elements include biodegradable fraction (fermentable), metals (except the part of the ferrous metals already removed by the magnetic elements by the conveyor belt (43) in case the optional 4^{th} step of the method was applied), plastic and glass. Perhaps in the product other materials are included, whose total mass is negligible compared to the mass of the already mentioned main elements. The total of the product is guided to the Stabilization Chamber (83). The Stabilization Chamber (83) is cylindrical in shape with a stable diameter. The main exit (102) (drawing 11) of the Stabilization Chamber (83), which leads the stabilized product to the next stage, includes a conical part (132) and then a cylindrical part (133) with stable diameter. The maximum diameter of the conical part (132) is equal to the diameter of the Stabilization Chamber (83), while the minimum diameter is equal to the diameter of the cylindrical part (133). The diameter of the conical part (132) is getting smaller according to the direction of the product towards the exit (102) from the Stabilization Chamber (83). The product exists from the opening situated in the cylindrical part (133).
The Stabilization Chamber (83) possesses a screw (103) and a stirrer (104) (drawings 15 & 16). The screw (103) and the stirrer (104) are placed axially to the center of a perpendicular section of the Stabilization Chamber (83). The screw (103) is longitudinal to the Stabilization Chamber (83) while the stirrer (104) has a length equal to the length of the Stabilization Chamber (83) that has the maximum stable section, reaching the initial conical part (132) of the exit (102) (dr. 11) of the Stabilization Chamber (83).
The screw (103) is of smaller diameter than the stirrer (104), and of greater length, reaching the cylindrical part (133) of the exit (102) (dr. 11) of the Stabilization Chamber (83). The screw (103) and the stirrer (104) are placed on an axis (105) and are coaxial, with the screw situated in the interior of the stirrer.
The axis (105) on one end is inside an adequate array (106) (drawing 15) that permits it to rotate freely. On the other end, the axis (105) has a pulley (107) (drawing 16) which is connected with special belts (108) (drawing 15) from adequate material which offers electrical insulation with a second pulley (109) (drawing 15) which rotates by a motor (110) (drawing 15). The axis (105) in any case is electrically insulated from the other part of the Stabilization Chamber (83). The electrical insulation is achieved with the use of proper insulating materials at the points where the axis (105) comes in contact with the rest of the body of the Stabilization Chamber (83). The screw (103) and the stirrer (104) are in electrical contact with the axis (105). For this reason the stirrer (104) has at its ends insulating material (111) (drawing 15) which comes in contact with the body of the Stabilization Chamber (83) and "scrapes" during its rotation the interior walls of the Stabilization Chamber (83) constructed of conductive material.

While the body of the Stabilization Chamber (83) is connected with the protective grounding (dr. 15), the axis (105) and by extension the screw (103) and the stirrer (104), since they are connected to each other, are connected with a positive high voltage load of controlled tension. When the product, which has a regulated percentage of humidity, comes into the interior of the Stabilization Chamber (83) and takes the space between the axis (105) and the Stabilization Chamber's (83) walls, it constitutes a conductor for the electron jump from the axis (105) to the walls of the Chamber (83), which are made of conductive material. This results to the breaking up of the DNA connections in the living organisms, like bacteria etc., and to the product's deliverance of germs and microorganisms.
The humidity regulation of the product inside the Stabilization Chamber (83) (dr. 11 & 15) is achieved by supplying treated liquids from the drainage tank (15) (dr. 3 & 4) of the liquid drainage collected initially at the 1^{st} step of the method in the reception funnel (1) for this reason and now they are reused, as mentioned before. The transfer of the liquids is done with a pump (137) (dr. 3) and the controlled injection into the interior of the Stabilization Chamber (83) by spraying nozzles (113) (drawing 15).

### 8^{th} Step: Exposure to UV radiation emission

Circumferentially on the walls of the Stabilization Chamber (83) but also on the upper axis (105) that bears the screw (103) and the stirrer (104), special arrays of UV radiation emission (114) are placed for the emission of UV radiation (drawings 15 and 16). The arrays of UV radiation emission (114), situated on the axis (105), are covered by special transparent material for their protection. The arrays of UV radiation emission (114) emit radiation in the area of 253,7nm, but also in the area of 185nm. The emission in the wavelength of 253,7nm has been proved to be the most effective wave length for the destruction of bacteria. The emission of radiation in the area of 185nm in connection to the oxygen existing inside the Stabilization Chamber (83) produces ozone which produces advanced oxidation of the polluting elements existing in the product. Furthermore, the ozone limits the odors which are eliminated. The UV radiation, to which the product is exposed when entering the Stabilization Chamber (83), hinders the growth of bacteria, by destroying the nucleus of their DNA, so that they cannot be reproduced. All the bacteria and the germs (viruses) are destroyed by the ultraviolet radiation, of course some more easily than others. This radiation is very effective against the bacteria as for many pathogenic microorganisms. Indicatively, we mention some of them: E. Coli, Legionella, Salmonella, Hepatitis, Poliovirus etc. Furthermore, the effect of the specific radiation wavelength can deactivate some microorganisms which then will multiply and become virulent. This phenomenon is observed in some bacteria (coliforms, shigellas).

The screw (103) has an opposite winding in relation to the axis (105) from the winding of the stirrer (104) (drawing 16). This results the screw (103) to push part of the product which enters the Stabilization Chamber (83) towards the next stage of the treatment while at the same time the stirrer (104) drags part of the product towards the opposite direction achieving this way a delay in the flow of the mass within the Stabilization Chamber (83). The delay is desirable because in this way the exposure of the product to the UV radiation is extended and the application of the method is optimized.

### 9^{th} Step (optional): Separation of glass, ferrous metallic elements, aluminium and plastic, by using a sorter of recyclable materials and channeling of materials to recycling.

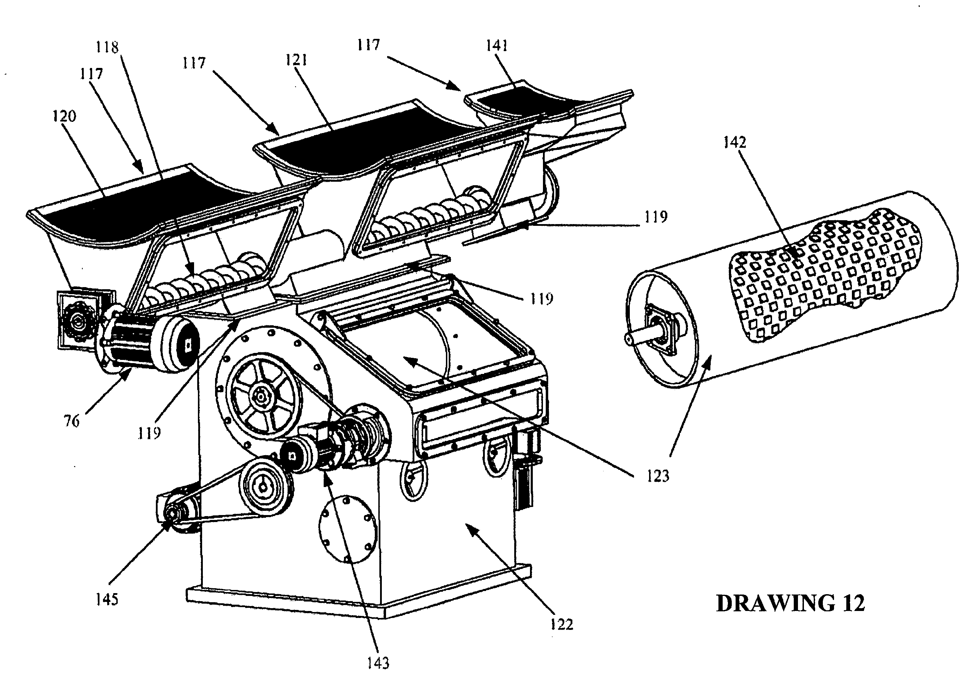

During the entrance of the product in the Stabilization Chamber (83), the heavy elements of the product are guided directly to the lower part of the Stabilization Chamber (83) because of gravity. Mainly at the lower part of the Stabilization Chamber (83) the metals, the plastic and the glass which have granular shape and bigger weight and diameter from the remaining elements of the product, are gathered. The lighter elements consisting mainly by biodegradable fraction of the product do not reach the lower part of the Stabilization Chamber (83) but are dragged by the screw (103) and guided to the next stage of the treatment which is the Microwave Chamber (116). For this part of the treatment which is optional there is reference at the end of the Description.
The stirrer (104) at its rotation drags the elements that are gathered in the lower part of the Stabilization Chamber (83) to the opposite direction from the one that the lighter elements are guided by the screw (103). At the lower part of the Stabilization Chamber (83) and in regular intervals between them there are openings (117) (drawing 12).
If this optional stage of the treatment is not to be followed, the Stabilization Chamber (83) does not need to have openings (117). Through these openings (117) and through the respective filters they have, the heavier elements are drawn by the rotating stirrer (104) to the lower part of the Stabilization Chamber (83) and are guided to the second screw (118) (drawing 12), which while it is single it has a thread of opposite direction. The first half of the second screw (118), starting from the motor (76) (drawing 12) that rotates it, guides the material from the left to the right while the second half makes the opposite.

Along this second screw (118) and on its lower part, there are three additional openings (119) through which the final selection of the elements takes place depending on their diameter.
This way, three separations of the product take place at the same time, depending on its granulometry, in connection to the openings (holes) of the three filters (120, 121 and 141).
In this way we achieve as described below, the removal of recyclable materials which are then separated and guided to recycling. This stage of the treatment can be used depending on the case and is not necessary for the application of the technology, as the material can avoid selection or recycling and go straight to the pelletization, or simply to be collected. In particular, the first filter (120) with the smaller openings is the one the product meets first, as it comes into the Stabilization Chamber (83), then it meets the second filter (121) with the slightly larger openings and thirdly it meets the third filter (141). with the largest openings
This way, from the first opening (117) (dr. 12) from left to right, and through the array of perforated metallic filter (120) with the specific holes that is has, exit the elements whose diameter is smaller than the holes of the first filter (120). As an example, and without the application of the technology of the method being restricted, the holes of the first metallic filter (120) could be 1 mm in diameter each. This way from the first filter (120) of the first opening (117) the elements of the product which are gathered in the lower part of the Stabilization Chamber (83) and have a diameter smaller or equal to 1mm are removed. These elements are mainly inert materials (e.g. dust, dirt) and are removed from the first opening (119).

From the second opening (117) from left to right, again through another perforated metallic filter (121) array with holes of larger diameter than the ones of the first filter (120) the elements whose diameter is larger than the diameter of the holes of the first filter (120), but smaller or equal to the diameter of the holes of the second filter (121) are exiting. As an example, and without restricting the application of the method, the holes of the second metallic filter (121) could be 5 mm in diameter each. In this way, from the second filter (121) of the second opening (117) the elements of the product gathered at the lower part of the Stabilization Chamber (83) having a diameter larger than 1mm, or the respective diameter of the holes of the first filter (120), and smaller or equal to 5mm are removed. The vast majority of the elements included in these removed through the second filter are mainly - because of their diameter - metals, glass and heavy plastics.

From the third perforated metallic filter (141) (drawing 12) of the third opening (117), are guided the elements of the product gathered on the lower part of the Stabilization Chamber (83) which were not shredded enough, and so they have a diameter larger than the diameter of the holes of the second filter (121). These elements are gathered for additional treatment.

The elements that are removed by passing from the second filter (121) are guided to the interior of the Special Sorter of Recyclable Materials (122) (dr. 12 & 14). The majority of these elements guided to selection and recycling through the second filter is glass, ferrous metals, aluminium and plastic. These elements can be removed together to be taken to special external units for the selection of recyclable material or to be guided to the Special Sorter of Recyclable Materials (122) of the present Municipal Solid Waste treatment system. The use of the Special Sorter of Recyclable Material (122) does not affect the application and by extension the effectiveness of the Municipal Solid Waste treatment method but constitutes an extra point of the system, which applies an optional step of the method, that if applied, effectuates a selection of the above recyclable materials, with gravity, magnetic elements, air draught and the use of electrostatic electricity.

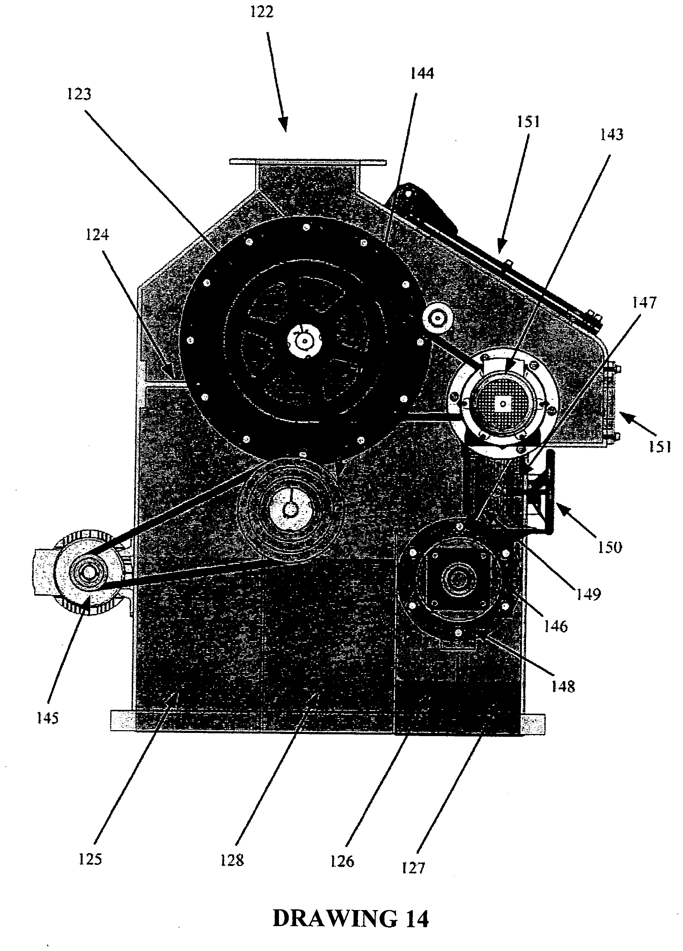

As depicted in drawing 12, the Special Sorter of Recyclable Materials (122) is connected to the second opening (119). Inside the sorter there is a rotating drum (123) (drawings 12 and 14) on which the elements coming from the second opening of the screw (118) are guided falling by gravity. The rotating drum (123) rotates by an array (143) of motor, belt, and pulley. The rotating dram drum (123) bears magnetic elements (142) (drawing 12) resulting to the magnetization of all the ferrous metals guided on it. At this point are magnetized all the ferrous metallic elements of small diameter, which had not been magnetized by the rotating axis (41) (dr. 10) as mentioned before, which is situated over the exit (49) of the horizontal measuring feeder (38) and bears magnetic elements (140) if the 4^{th} and optional step of the method was applied.
The other elements like glass, plastic, and nonferrous metals (aluminium) are not getting magnetized and continue falling by gravity inside the Special Sorter of Recyclable Materials (122). The ferrous metals because of the magnetic attraction they get from the rotating drum (123) are getting attached to its surface and rotate with it. In parallel with the rotating drum (123) there is a special scraper shaft (124) which is tangentially in contact with the rotating drum (123). The scraper shaft (124) is tangentially in contact with the drum (123) in such a way that it does not hinder its rotation. The rotation of the magnetized ferrous metallic elements continuous until the point they meet the scraper shaft (124) which detaches them from the magnetic rotating drum (123) and guides them inside the collector (125) of the ferrous metallic elements. The ferrous metallic elements are gathered in the collector (125) and are removed from there. Inside the special Sorter of Recyclable Materials (122) and vertically to the falling direction of the nonferrous elements, that is horizontally, an air draught is created that blows them depending on their weight. The air draught is created by a centrifugal pump (144) which is rotating by an array (145) of a motor, belt and pulley. The air draught created by the centrifugal pump (144) blows the light elements, in their majority aluminium and plastic, towards the rotating electrostatic drum (146). The rotating electrostatic drum (146) rotates by an array of motors (147). The aluminium inside the product has mainly the form of very thin and light leaves. The aluminium along with the plastic is guided by the air draught to the rotating electrostatic drum (146). The rotating electrostatic drum (146) draws the plastic elements which are attached to the surface of the drum (146). The aluminium is not drawn by the rotating electrostatic dram (146) resulting to its fall inside the collector (126) for aluminium from where it is later removed. The plastic, which is attached to the surface of the rotating electrostatic drum (146), rotates with the rotating electrostatic drum (146) and is guided to the collector (127) of plastics, from where it is later removed. The detachment of the plastic elements from the surface of the rotating electrostatic drum (146) takes place with the help of an adequate metallic scraper array (148) which is tangentially in contact with the surface of the rotating electrostatic drum (146), without hindering its free rotation. For facilitating the light elements (aluminium, plastic) to be guided on the surface of the rotating electrostatic drum (146) there is a metallic guidance array (149). The metallic array (149) is placed on a proper point, while the angle that it forms with the rotating electrostatic drum (146) is regulated in order to achieve its optimum use. The regulation of the metallic array (149) is achieved through external rotating regulators (150). The heavier elements rotating in the incoming material to the Special Sorter of Recyclable Materials (122), which are mainly glass, are not drawn by the current of air created by the centrifugal pump (144) and fall by gravity into the collector (128) of glass, from where they are later removed. With the above mentioned way the separation of the elements coming inside the Special Sorter of Recyclable Materials (122) is achieved, in separate collector (128) for glasses, collector (125) for ferrous metallic elements, collector (126) for aluminium and collector (127) for plastics. The Special Sorter of Recyclable Materials (122) has adequate access hatches (151).

If this step of selection is not applied, since it is optional for the application of the method, as mentioned before, then all the materials are guided directly to the 10^{th} step which is also optional and is the exposure of the elements to a microwave radiation, or to the 11^{th} step, also optional, which is pelletization, or they are simply removed.

10^{th} Step (optional): Exposure of lighter elements to microwave radiation. Further sterilization of the product takes place in the Microwave Chamber (116). This stage of the treatment guarantees the production of a safe product, completely free of infectious factors. This stage is not necessary for the effectiveness of the method and the system but is optional for the optimization of the application of the method. This stage helps the Stabilization Chamber (83) and guarantees the safety of the final product produced by the system.

This stage refers to the lighter elements, consisted of, in their majority, biodegradable fraction of the product and which do not reach the lower part of the Stabilization Chamber (83), but are dragged by the screw (103) and guided to the microwave treatment. On the contrary metals, plastic and glass which has a granular shape and larger weight but also larger diameter from the rest of the elements of the product, are gathered mainly on the lower part of the Stabilization Chamber (83) and from there they are guided - optionally - to the Special Sorter of Recyclable Materials (122) as presented immediately before.
This way, the stabilized product free of infectious and pathogenic organisms is transferred by the screw (103) to the exit of the Stabilization Chamber (83). From there it enters by gravity to the following, optional, stage of treatment which is the special Microwave Chamber (116). Therefore, the lighter elements, with the rotation of the screw (103) are exiting from the Stabilization Chamber (83) and entering the Microwave Chamber (116), which is the continuation of the Stabilization Chamber (83).

In the Microwave Chamber (116) (drawings 11 and 15) there are arrays of microwave emission (115). Through these arrays of microwave emission (115) the product entering the Microwave Chamber (116) is submitted to high energy radiation which is properly coordinated to be absorbed by water molecules (humidity) existing in the product. This results to the additional sterilization of the mass of the product existing inside the Microwave Chamber (116).

### 11^{th} Step (optional): Array of final stabilization, volume and mass decrease (131) (Pelletizing)

Then the product enters the Final Stabilization Array (131) for Decrease of Volume and Mass. The Final Stabilization Array (131) for Decrease of Volume and Mass is connected to the Microwave Chamber (116) -since this optional stage of the method will be applied - in such a way that the product at its course from the Stabilization Chamber (83) to the Microwave array (116) and from there to the Stabilization Array (131), is always entering smoothly without being blocked, such as by gravity, by air transfer, or by screw, etc.
At this stage of treatment, the product is compressed aiming to its additional decrease of volume and mass. This is achieved with the use of one pelletization machine known to the modern level of technology treating the product and converting it to pellets of high density. The product after passing from the pelletization machine and after we receive the final dehydrated and stabilized product is guided to the packaging machine. The material during its stay in the interior of the pelletization machine is submitted to conditions of pressure and temperature for enough time for the final stabilization of the product to be achieved and the discharge of the microbial load dangerous for public health.

Additionally, the system possesses in many points washing and disinfection arrays (130) (dr. 2, 15, 19 and 20) of the partial units, arrays and equipment. The washing arrays are necessary for the systematic disinfection of the complex in order to reduce the microbial load and eliminate the odors.

All the previously described system is constructed in such a way that it is fully sealed against drainage, leakage and gasses or odors.

The operation of the complex is fully automated and electronically controlled. No human presence is necessary in the stages of execution of the treatment and exploitation, only for the regulation of the operational parameters, which can be only the initial by the constructor. In this case, the human presence is restricted to the programming of new parameters through the central control board. In this way the manual interventions are minimized to all the operational stages and therefore the exposure of the workers' health to infection risks.
The operation of the complex is ecologically safe as there are no solid or liquid residues while clean air is emitted to the atmosphere.

The complex of the present invention is transported inside containers and is internally adapted and externally bears the proper openings and connection arrays. In this way the complex is ready for operation at any moment, needing only its transportation to the desired point with the containers which can be transferred anywhere and applies the present method for the waste treatment.

Finally, the containers which can be incorporated on the tractor of the system of the present invention can either be detachable of the frame of the tractor or stable on its frame.

## Claims

1. A system for treatment of Municipal Solid Waste, comprising a reception funnel (1) for entry of the Municipal Solid Waste into the system, under which there is a first conveyor for transfer of the waste towards a Special Opening Unit (19) of Municipal Solid Waste packages and a Special Homogenization Unit (28) of the Municipal Solid Waste, while outside the reception funnel (1) there is at least one separation system (17); a vertical pad elevator (31); a Horizontal Measuring Feeder (38) for controlling the feeding flow to a Low Frequency Pulsator (39) for pulverization of material; a High Frequency Pulsator (68); a Transfer system, configured to transfer the product from the High Frequency Pulsator (68) to a Stabilization Chamber (83), wherein the High Frequency Pulsator (68) is configured to develop pulses of higher frequency than the Low Frequency Pulsator (39), **characterized in that**:
i) The first conveyor is a first chain conveyor (5), which is on the lower part of the reception funnel (1), so that under the first chain conveyor (5) and along the reception funnel (1) there is a drainage tank (15) with at least one pump (16) for thick fluids;
ii) The Special Opening Unit (19), which comprises at least: one motor (23), a pulley and belt array (24) or an electro-reducer and a rotating axis (21), parallel to the first chain conveyor (5) and at a regulated distance from it, which bears cutting discs (22) from high resistance material, with regulated rotation speed and at distance from the first chain conveyor (5);
iii) The Special Homogenization Unit (28), which comprises at least: an electric motor (36) and arrays of belts - pulleys or electric reducer (79), rotating axis (29), which is horizontal and parallel to the level of the first chain conveyor (5) and at a regulated distance from it and bears metal plates (30), bearings (35) and horizontal supporting axis (33), parallel to the rotating axis (29), while lateral supports (34) are vertically placed on either side of the horizontal supporting axis (33), inside vertical guides (37) and coil springs (32), vertical to the horizontal supporting axis (33), permitting the vertical movement of the Special Homogenization Unit (28);
iv) The vertical pad elevator (31) has entrance and exit (48), so that the entrance is connected either to the first chain conveyor (5) or to a horizontal conveyor (136), while the exit (48) is connected with the entrance (47) of the Horizontal Measuring Feeder (38);
v) The Horizontal Measuring Feeder (38) comprises: a metallic frame (42), a lid (44), a bottom (45), a conveyor belt (43), pads (51) with equal length to the width of the conveyor belt (43) and the height is such that there is no space between the bottom (45) and the pads (51) and two rotating axes (40, 41), wherein one of the rotating axes (40) is rotatable by an electric motor and a reducer (134) and is configured to move the conveyor belt (43), while the pads (51) are configured to drive the product towards an exit (49), while the other rotating axis (41) is configured to rotate freely, while the exit (49) is connected with an entrance (50) of the Low Frequency Pulsator (39);
vi) The Low Frequency Pulsator (39) situated inside a robust metallic construction (55), which has the entrance opening (50) on its upper part, where an exit of a feeding funnel (52) is connected and an exit opening (67) on the lower part, is comprising: a rotating axis (58), configured to come vertically from the center of at least two metallic supporting discs (59), which bear pulse elements (61) between them, fastened with a perforated rotating axis (62) on cylindrical axes (60), parallel to the rotating axis (58), while on the lower part of the metallic construction (55) there is a metallic array of horizontal control elements (63) of the volume, which are situated in determined distances between them, configured to create openings (64), with the exit opening (67) of the material to a second chain conveyor (66), connected to the High Frequency Pulsator (68);
vii) The High Frequency Pulsator (68), situated within a robust metallic construction (85), is comprising a rotating axis (88), configured to come vertically from the center of at least two metallic supporting discs (89), which are parallel to each other and vertical to the rotating axis (88) and bear between them pulse elements (91), fastened at a distance determined by a perforated axis (92) on cylindrical axes (90), which are parallel to the rotating axis (88), whereas the number of the cylindrical axes (90) and also the number of the pulse elements (91) of each cylindrical axis (90) is equal or higher than the number of pulse elements (62) and the cylindrical axes (60) of the Low Frequency Pulsator (39), and whereas on the lower part of the metallic construction (85), there is a metallic array of control elements (93) of the volume, with openings (94) with smaller dimensions than the openings (64) of metallic array of horizontal control elements (63) of the Low Frequency Pulsator (39);
viii) The Transfer system is an Air Transfer system, which is comprising: a filter array (84), a second air centrifugal pump (96) of high pressure and sub-pressure with an entry (97), which is connected with the exit of the Low Frequency Pulsator (68) through a tube (98) and an exit (100), which is connected through tubing (101) with the rear part of the Stabilization Chamber (83), where the second air centrifugal pump (96) is configured to create an intense air draught with a direction from the High Frequency Pulsator (68) towards the Stabilization Chamber (83) inducing the violent transfer of the product to it and a first air centrifugal pump (81) of pressure and sub-pressure, connected to the Stabilization Chamber (83) and configured to remove from it the excess air and the surplus humidity created in the interior of the Stabilization Chamber (83) through a filter array (84);
ix) The Stabilization Chamber (83) is cylindrical in shape with stable diameter, grounded, with openings (117) on the lower part and an exit (102), which includes a conical part (132) and a cylindrical part (133) with stable diameter, whereby the Stabilization Chamber (83) is comprising: circumferentially on the walls of the Stabilization Chamber (83) but also on an axis (105), arrays of UV radiation emission (114), configured to emit radiation in the area of 253,7nm but also in the area of 185nm, so that those, placed on the axis (105), are covered by a special transparent material for their protection, a screw (103) and a stirrer (104), placed axially to the center of the vertical cross-section of the Stabilization Chamber (83), where the stirrer (104) is of equal length to the length of the Stabilization Chamber (83), which has maximum stable cross-section, configured to reach until the beginning of the conical part (132) of the exit (102) of the Stabilization Chamber (83), while the screw (103) is longitudinal to the Stabilization Chamber (83) and is of smaller diameter to the stirrer (104) and of greater length, configured to reach until the cylindrical part (133) of the exit (102) of the Stabilization Chamber (83),the screw (103) and the stirrer (104) are placed coaxially on the axis (105), insulated from the other part of the Stabilization Chamber (83), wherein the screw (103) is in the interior of the stirrer (104) and has an opposite winding in relation to the axis (105) from the winding of the stirrer (104), where the axis (105) has one end free, and on the other bears a pulley (107), which is connected with belts (108) from material for electrical insulation with a second pulley (109), which is rotatable by a motor (110), the screw (103) and the stirrer (104) are in electrical contact with the axis (105) and the stirrer (104) has at its ends an insulating material (111), which is in contact with the body of the Stabilization Chamber (83) and is configured to scrape during rotation the interior walls of the Stabilization Chamber (83), constructed of conductive material and the body of the Stabilization Chamber (83) is connected to protective grounding, while the axis (105), the screw (103) and the stirrer (104) are connected to positive high voltage load of controlled tension, while at least one pump (137) is configured to transfer the treated liquids from the drainage tank (15) in the interior of the Stabilization Chamber (83) with spraying nozzles (113).

2. A system for treatment of Municipal Solid Waste, according to claim 1, **characterized in that** the axis (41) of the Horizontal Measuring Feeder (38) over the exit (49), bears magnetic elements (140) for selection of ferrous metals, therefor on the bottom (45) of the Horizontal Measuring Feeder (38) is situated a second exit (139) for transfer of the selected ferrous metals to a separate collection.

3. A system for treatment of Municipal Solid Waste, according to claims 1 and 2, **characterized in that** at the lower part of the Stabilization Chamber (83) in regular intervals between them there are openings (117), while each opening (117) has a metallic filter (120, 121 and 141) with holes, whereby the first metallic filter (120) is with smallest holes, the second metallic filter (121) is with larger holes and the third metallic filter is with largest holes and the Stabilization Chamber (83) bears in its interior a second screw (118) with a thread of opposite direction from the edges towards the center, which is configured to lead the material from the edges to the center, while the lower part of the second screw (118) has three openings (119) for final selection of elements.

4. A system for treatment of Municipal Solid Waste, according to claims 1 to 3, **characterized in that** a Special Sorter of Recyclable Materials (122) is configured to be used for separation of glass, ferrous metallic elements, aluminium and plastic is connected to the central opening (119) wherein inside the Special Sorter of Recyclable Materials (122) there is a rotating drum (123) with magnetic elements (142), a special scraper shaft (124), which is tangentially in contact with the rotating drum (123), a collector (125) for the ferrous metallic elements and a centrifugal pump (144), configured to create an air draught and to guide the light elements, to the rotating electrostatic drum (146), with a metallic scraper array (148) for detachment of the plastic, which is tangentially in contact with the rotating electrostatic drum (146), under regulated angle through external rotating regulators (150), with a collector (127) for plastics, collector (126) for aluminium and collector (128) for glass.

5. A system for treatment of Municipal Solid Waste, according to claims 1 to 4, **characterized in that** a Microwave Chamber (116) with microwave emission arrays (115) is situated on the exit of the Stabilization Chamber (83).

6. A system for treatment of Municipal Solid Waste, according to claims 1 to 5, **characterized in that** a Final Stabilization Array (131) for Decrease of Volume and Mass is connected to the Microwave Chamber (116).

7. A system for treatment of Municipal Solid Waste, according to claims 1 to 6, **characterized in that** the second chain conveyor (66) is of closed type and has a rotating axis (69), (70) at both ends, wherein the rotating axis (69), situated under the Low Frequency Pulsator (39), is connected to an electric motor (71) through a reducer (72), configured to transfer motion to a chain (73) of the second chain conveyor (66), while the rotating axis (70), situated over the High Frequency Pulsator (68), is placed in guides (74), which are configured to permit the horizontal movement regarding the movement level of the second chain conveyor (66) and each rotating axis (69), (70) includes at each end serrated discs (75), vertical to the edges of the rotating axes (69), (70), which are equal in number to the chains (73) of the chain conveyor (66), whereby each serrated disc (75) has on its surface recesses, on which each chain (73) is configured to rest and metallic sheets (77) are placed vertical to the chains (73) at regular intervals and at distance to each other from around 40 cm to 80 cm, where on each metallic sheet (77) and to the interior of the second chain conveyor (66) is placed a longitudinal strip of material (78) with equal length to the length of the metallic sheet (77), wherein through the second chain conveyor (66) a violent artificial flow of air is creatable by the second air centrifugal pump (96).

8. A system for treatment of Municipal Solid Waste, according to claims 1 to 7, **characterized in that** the reception funnel (1) has windproof sheets (2), a photocell (4) for material level control, a waste block system (138) and a visiting manhole (3).

9. A system for treatment of Municipal Solid Waste, according to claims 1 to 8, **characterized in that** the first chain conveyor (5) is equipped with a system for flow control to avoid jamming in the Special Opening Unit (19), with regulated movement speed and is comprising at least one chain (6) and a sliding surface (7) which is compact and has a gradient or is perforated and metallic and bears on both sides of its edges two cylindrical axes (8) with serrated discs (9), which are equal in numbers to the chains (6), whereby in their perimeter the serrated discs (9) have recesses (10) of adequate size, inside which every chain (6) is rested, wherein the first chain conveyor (5) is extended under the special opening unit (19) and under the special homogenization unit (28) and is configured to deposit the transferred material directly to the entrance of the vertical pad elevator (31) or to the horizontal conveyor (136).

10. A system for treatment of Municipal Solid Waste, according to claims 1 to 9, **characterized in that** the pump (16) for thick fluids is inside the drainage tank (15) and is configured to lead the thick fluids from the drainage tank (15) to the separation system (17), which is configured to lead the solids inside the reception funnel (1) or to an exterior collector and the fluids through pumps (137) to at least one liquid drainage storage tank (18), which has inside a filter of active carbon (112) for the cleaning of the fluids, while the spraying nozzles (113) are arranged to spray the liquids, through at least one additional filter (65) and adequate tubing, in the reception funnel (1), the Low Frequency Pulsator (39), the High Frequency Pulsator (68) and the Stabilization Chamber (83).

11. A system for treatment of Municipal Solid Waste, according to claims 1 to 10, **characterized in that** the Special Opening Unit (19), the Special Homogenization Unit (28), the metallic construction (55) of the Low Frequency Pulsator (39), the second chain conveyor (66), the metallic construction (85) of the High Frequency Pulsator (68), the feeding funnel (52) and the Special Sorter of Recyclable Materials (122), have protection lids (25), access hatches (26, 53, 56, 80, 86, 151), windows (27, 54, 57, 80, 87), visual control systems (20) and washing and sterilization arrays (130).

## Patentansprüche

1. Ein System zur Aufbereitung von Hausmüll, das einen Aufnahmetrichter (1) zum Eintritt des Siedlungsabfalls in das System umfasst, unter dem sich ein erster Förderer für die Übertragung der Abfälle zu einer speziellen Öffnungseinheit (19) von Siedlungsabfallverpackung und eine spezielle Homogenisierungseinheit (28) des Siedlungsabfalls befindet, während außerhalb des Aufnahmetrichters (1) mindestens ein Trennsystem (17); einen vertikalen Aufzug (31); eine horizontale Messzuführung (38) zum Steuern des Zufuhrstroms zu einem Niederfrequenzpulsator (39) zum Pulverisieren des Materials; einen Hochfrequenzpulsator (68); ein Übertragungssystem, das konfiguriert ist, um das Produkt vom Hochfrequenzpulsator (68) zu einer Stabilisierungskammer (83) zu übertragen, vorhanden sind, wobei der Hochfrequenzpulsator (68) konfiguriert ist, um Impulse mit höherer Frequenz als die Frequenz des Niederfrequenzpulsators (39) zu entwickeln; **gekennzeichnet dadurch, dass**:
i) der erste Förderer ein erster Kettenförderer (5) ist, der sich im unteren Teil des Aufnahmetrichters (1) befindet, so dass unter dem ersten Kettenförderer (5) und entlang des Aufnahmetrichters (1) ein Abflusstank (15) mit mindestens einer Pumpe (16) für dicke Flüssigkeiten vorhanden ist;
ii) die spezielle Öffnungseinheit (19), die mindestens einen Motor (23), eine Scheiben- und Gürtelanordnung (24) oder ein elektrisches Reduzierstück und eine Drehachse (21) parallel zum ersten Kettenförderer (5) und in einem geregelten Abstand davon umfasst, die Schneidscheiben (22) aus hochbeständigem Material mit geregelter Drehzahl und in Abstand vom ersten Kettenförderer (5) trägt;
iii) die spezielle Homogenisierungseinheit (28), die mindestens Folgendes umfasst: einen Elektromotor (36) und eine Scheiben- und Gürtelanordnung oder ein elektrisches Reduzierstück (79), eine Drehachse (29), die horizontal und parallel zum Niveau der erster Kettenförderer (5) und in einem geregelten Abstand davon ist, und Metallplatten (30), Lager (35) und horizontale Stützachse (33) parallel zur Drehachse (29) trägt, während seitliche Stützen (34) vertikal auf beiden Seiten der horizontalen Stützachse (33) innerhalb vertikaler Führungen (37) und Schraubenfedern (32) senkrecht zur horizontalen Stützachse (33) angeordnet sind, um die vertikale Bewegung der speziellen Homogenisierungseinheit (28) zu ermöglichen;
iv) der vertikale Aufzug (31) einen Ein- und Ausgang (48) hat, so dass der Eingang entweder mit dem ersten Kettenförderer (5) oder mit einem horizontalen Förderer (136) verbunden ist, während der Ausgang (48) mit dem Eingang (47) der horizontalen Messzuführung (38) verbunden ist;
v) der horizontale Messzuführung (38) das Folgende umfasst: einen Metallrahmen (42), einen Deckel (44), einen Boden (45), einen Förderer (43), Beläge (51) mit der gleichen Länge wie die Breite des Förderers (43), mit einer solcher Höhe, dass zwischen dem Boden (45) und den Belägen (51) und den zwei rotierenden Achsen (40, 41) kein Raum vorhanden ist, wobei eine der rotierenden Achsen (40) durch einen Getriebemotor (134) angetrieben wird und ist konfiguriert, das Förderband (43) zu bewegen, während die Beläge (51) konfiguriert sind, um das Produkt in Richtung eines Ausgangs (49) zu treiben, wobei die andere Drehachse (41) konfiguriert ist, um sich frei zu drehen, wobei der Ausgang (49) mit einem Eingang (50) des Niederfrequenzpulsators (39) verbunden ist;
vi) der Niederfrequenzpulsator (39), der sich in einem robusten Metallgehäuse (55) befindet, dessen oberer Teil die Eintrittsöffnung (50) aufweist, an der ein Ausgang eines Zuführtrichters (52) angeschlossen ist, und deren unteren Teil eine Austrittsöffnung (67) aufweist, das Folgende umfasst: eine Drehachse (58), die so konfiguriert ist, dass sie vertikal von der Mitte mindestens zweier metallischer Stützscheiben kommt (59), die Impulselemente (61) zwischen sich tragen, die mit einer perforierten Drehachse (62) an zylindrischen Achsen (60) parallel zur Drehachse (58) verbunden sind, wobei am unteren Teil des Metallgehäuses (55) eine metallische Anordnung horizontaler Volumensteuerelemente (63) vorhanden ist, die sich in bestimmten Abständen befinden und so konfiguriert sind, dass sie Öffnungen (64) erzeugen, wobei die Austrittsöffnung (67) des Materials zu einem zweiten Kettenförderer (66) führt, der mit dem Hochfrequenzpulsator (68) verbunden ist;
vii) der Hochfrequenzpulsator (68), der sich in einem robusten Metallgehäuse (85) befindet, eine Drehachse (88) umfasst, die so konfiguriert ist, dass sie vertikal von der Mitte mindestens zweier metallischer parallel zueinander und senkrecht zur Drehachse (88) stehender Stützscheiben (89), die zwischen sich Impulselemente (91) tragen, die in einem durch eine Lochachse (92) bestimmten Abstand an Zylinderachsen (90) befestigt sind, die parallel zur Drehachse (90) verlaufen 88), wobei die Anzahl der Zylinderachsen (90) und auch die Anzahl der Impulselemente (91) jeder Zylinderachse (90) gleich oder höher als die Anzahl der Impulselemente (62) und der Zylinderachsen (60) des Niederfrequenzpulsators (39) ist und wobei sich im unteren Teil des Metallgehäuses (85) eine metallische Anordnung von Volumensteuerelementen (93) befindet, dessen Öffnungen (94) mit kleineren Abmessungen als die Öffnungen (64) der metallischen Anordnung horizontaler Steuerelemente (63) des Niederfrequenzpulsators (39) sind;
viii) das Übertragungssystem ein Luftübertragungssystem ist, das Folgendes umfasst: eine Filteranordnung (84), eine zweite Luftzentrifugalpumpe (96) mit hohem Druck und Unterdruck mit einem Eingang (97), der mit dem Ausgang des Niederfrequenzpulsators (68) durch ein Rohr (98) und einen Ausgang (100) verbunden ist, welcher Ausgang durch ein Rohr (101) mit dem hinteren Teil der Stabilisierungskammer (83) verbunden ist, wo die zweite Luftzentrifugalpumpe (96) so konfiguriert ist, dass sie einen intensiven Luftzug mit einer Richtung vom Hochfrequenzpulsator (68) zur Stabilisierungskammer (83) erzeugt, der die heftige Übertragung des Produkts auf diese und eine erste Luftkreiselpumpe (81) von Druck und Unterdruck induziert, das mit der Stabilisierungskammer (83) verbunden ist und so konfiguriert ist, dass die überflüssige Luft und die überschüssige Feuchtigkeit, die im Inneren der Stabilisierungskammer (83) durch eine Filteranordnung (84) erzeugt werden, von dieser entfernt werden;
ix) die Stabilisierungskammer (83) eine zylindrische Form mit stabilem Durchmesser hat, geerdet ist, mit Öffnungen (117) am unteren Teil und einem Ausgang (102) ausgestattet ist, welcher Ausgang einen konischen Teil (132) und einen zylindrischen Teil (133) mit stabiler Durchmesser hat, wobei die Stabilisierungskammer (83) das Folgende umfasst: in Umfangsrichtung an den Wänden der Stabilisierungskammer (83), aber auch auf einer Achse (105), Anordnungen der UV-Strahlungsemission (114), die so konfiguriert sind, dass sie Strahlung im Bereich von 253.7 nm, aber auch im Bereich von 185 nm, ausstrahlen, so dass diejenigen, die auf der Achse (105) angeordnet sind, zu ihrem Schutz von einem speziellen transparenten Material bedeckt sind, eine Schraube (103) und ein Rührer (104), die axial zur Mitte des vertikalen Querschnitts der Stabilisierungskammer (83) angeordnet sind, wobei der Rührer (104) gleich lang ist wie die Länge der Stabilisierungskammer (83), die einen maximalen stabilen Querschnitt aufweist und so konfiguriert ist, dass sie bis zum Beginn des konischen Teils (132) des Ausgangs (102) der Stabilisierungskammer (83) reicht, wobei die Schraube (103) in Längsrichtung zur Stabilisierungskammer (83) steht und einen kleineren Durchmesser zum Rührer (104) und eine größere Länge hat und so konfiguriert ist, dass sie bis zum zylindrischen Teil (133) des Ausgangs (102) der Stabilisierungskammer (83) reicht, die Schraube (103) und der Rührer (104) sind koaxial auf der Achse (105) angeordnet, isoliert von dem anderen Teil der Stabilisierungskammer (83), wobei sich die Schraube (103) im Inneren des Rührers (104) befindet und eine entgegengesetzte Wicklung in Bezug auf die Achse (105) von der Wicklung des Rührers (104) hat, wobei die Achse (105) ein Ende frei hat und auf der anderen eine Riemenscheibe (107) trägt, die mit einem Riemen (108) aus elektrisch isolierenden Material mit einer zweiten Riemenscheibe (109) verbunden ist, die durch einen Motor (110) drehbar ist, die Schraube (103) und der Rührer (104) stehen in elektrischem Kontakt mit der Achse (105) und der Rührer (104) hat an seinen Enden ein Isoliermaterial (111), das mit dem Körper der Stabilisierungskammer (83) in Kontakt steht und so konfiguriert ist, dass es während der Drehung die Innenwände der Stabilisierungskammer (83) kratzt, wobei die Wände aus leitendem Material bestehen und der Körper der Stabilisierungskammer (83) mit einer Schutzerdung verbunden ist, wobei die Achse (105), die Schraube (103) und der Rührer (104) mit einer positiven Hochspannungslast mit kontrollierter Spannung verbunden sind, wobei mindestens eine Pumpe (137) so konfiguriert ist, dass sie die behandelten Flüssigkeiten mit Sprühdüsen (113) aus dem Abflusstank (15) im Inneren der Stabilisierungskammer (83) fördert.

2. Ein System zur Aufbereitung von Hausmüll nach Anspruch 1, **dadurch gekennzeichnet, dass** die Achse (41) der horizontalen Messzuführung (38) über dem Ausgang (49) magnetische Elemente (140) zur Auswahl von Eisenmetallen hat, daher befindet sich am Boden (45) der horizontalen Messzuführung (38) ein zweiter Ausgang (139) zur Übertragung der ausgewählten Eisenmetalle in einen separaten Kollektor.

3. Ein System zur Aufbereitung von Hausmüll nach den Ansprüchen 1 und 2, **dadurch gekennzeichnet, dass** im unteren Teil der Stabilisierungskammer (83) in regelmäßigen Abständen Öffnungen (117) vorhanden sind, wobei jede Öffnung (117) ein Metallfilter (120, 121 und 141) mit Löchern hat, wobei der erste Metallfilter (120) mit kleinsten Löchern, der zweite Metallfilter (121) mit größeren Löchern und der dritte Metallfilter mit größten Löchern ist und die Stabilisierungskammer (83) in ihrem Inneren eine zweite Schraube (118) mit einem Gewinde entgegengesetzter Richtung von den Kanten zur Mitte hat, welche Schraube so konfiguriert ist, dass sie das Material von den Kanten zur Mitte führt, während der untere Teil der zweiten Schraube (118) drei Öffnungen (119) für die endgültige Auswahl der Elemente hat.

4. Ein System zur Aufbereitung von Hausmüll nach den Ansprüchen 1 bis 3, **dadurch gekennzeichnet, dass** ein spezieller Sortierer von recycelbaren Materialien (122) so konfiguriert ist, dass er zur Trennung von Glas, Eisenmetallelementen, Aluminium und Kunststoff verwendet wird und mit der zentralen Öffnung (119) verbunden ist, wobei sich innerhalb des Spezialsortierers von recycelbaren Materialien (122) die folgende Komponenten befinden: eine rotierende Trommel (123) mit magnetischen Elementen (142), eine spezielle Abstreifwelle (124), die tangential mit der rotierenden Trommel (123) in Kontakt steht, einen Kollektor (125) für die Eisenmetallelemente und eine Zentrifugalpumpe (144), die konfiguriert ist, um einen Luftzug zu erzeugen und die Lichtelemente zu der rotierenden elektrostatischen Trommel (146) zu führen, mit einer Metallabstreiferanordnung (148) zum Ablösen der Kunststoffe, der tangential mit der rotierenden elektrostatischen Trommel (146) in Kontakt steht, unter geregeltem Winkel durch externe rotierende Regler (150), mit einem Kollektor (127) für Kunststoffe, Kollektor (126) für Aluminium und Kollektor (128) für Glas.

5. Ein System zur Aufbereitung von Hausmüll nach den Ansprüchen 1 bis 4, **dadurch gekennzeichnet, dass** sich am Ausgang der Stabilisierungskammer (83) eine Mikrowellenkammer (116) mit Mikrowellenemissionsanordnungen (115) befindet.

6. Ein System zur Aufbereitung von Hausmüll nach den Ansprüchen 1 bis 5, **dadurch gekennzeichnet, dass** ein Endstabilisierungsanordnung (131) zur Verringerung von Volumen und Masse mit der Mikrowellenkammer (116) verbunden ist.

7. Ein System zur Aufbereitung von Hausmüll nach den Ansprüchen 1 bis 6, **dadurch gekennzeichnet, dass** der zweite Kettenförderer (66) vom geschlossenen Typ ist und an beiden Enden eine Drehachse (69), (70) aufweist, wobei die Drehachse (69), das sich unter dem Niederfrequenzpulsator (39) befindet, über ein Reduzierstück (72) mit einem Elektromotor (71) verbunden ist, das konfiguriert ist, um eine Bewegung auf eine Kette (73) des zweiten Kettenförderers (66) zu übertragen, während die Drehachse (70), die sich über dem Hochfrequenzpulsator (68) befindet, in Führungen (74) angeordnet ist, die so konfiguriert sind, dass sie die horizontale Bewegung in Bezug auf das Bewegungsniveau des zweiten Kettenförderers (66) und jeder Drehachse (69), (70) ermöglichen, an jedem Ende gezackte Scheiben (75) senkrecht zu den Kanten der Drehachsen (69), (70) hat, deren Anzahl den Ketten (73) des Kettenförderers (66) entspricht, wobei jede gezackte Scheibe (75) auf ihrer Oberfläche Aussparungen aufweist, auf denen jede Kette (73) zum Ruhen konfiguriert ist und Metallbleche (77) in regelmäßigen Abständen und in einem Abstand von etwa 40 cm bis 80 cm zu den Ketten (73) montiert sind, wobei auf jedem Metallblech (77) und im Inneren des zweiten Kettenförderers (66) ein Längsstreifen (78) mit gleicher Länge wie die Länge des Metallblechs (77) angeordnet ist, wobei durch den zweiten Kettenförderer (66) ein heftiger künstlicher Luftstrom durch die zweite Luftzentrifugalpumpe (96) erzeugt werden kann.

8. Ein System zur Aufbereitung von Hausmüll nach den Ansprüchen 1 bis 7, **gekennzeichnet dadurch, dass** der Aufnahmetrichter (1) winddichte Bleche (2), eine Fotozelle (4) zur Materialstandkontrolle, ein Abfallblocksystem (138) und ein Zugangsschacht (3) hat.

9. Ein System zur Aufbereitung von Hausmüll nach den Ansprüchen 1 bis 8, **gekennzeichnet dadurch, dass** der erste Kettenförderer (5) mit einem System zur Durchflussregelung ausgestattet ist, um ein Verklemmen in der speziellen Öffnungseinheit (19) zu vermeiden, mit geregelter Bewegungsgeschwindigkeit, und mindestens eine Kette (6) und eine Gleitfläche (7) umfasst, die kompakt ist und einen Gradienten aufweist oder perforiert und metallisch ist und auf beiden Seiten ihrer Kanten zwei zylindrische Achsen (8) mit gezackten Scheiben (9) trägt, dessen Anzahl gleich den Ketten (6) ist, wobei die gezackten Scheiben (9) in ihrem Umfang Aussparungen (10) von ausreichender Größe aufweisen, in denen jede Kette (6) ruht, wobei der erste Kettenförderer (5) unter der speziellen Öffnungseinheit (19) und unter der speziellen Homogenisierungseinheit (28) verlängert und konfiguriert ist, um das übertragene Material direkt zum Eingang des vertikalen Aufzugs (31) oder zum horizontalen Förderer (136) abzulegen.

10. Ein System zur Aufbereitung von Hausmüll nach den Ansprüchen 1 bis 9, **dadurch gekennzeichnet, dass** sich die Pumpe (16) für dicke Flüssigkeiten im Abflusstank (15) befindet und so konfiguriert ist, dass sie die dicken Flüssigkeiten aus dem Abflusstank (15) zu dem Trennsystem (17) leitet, das konfiguriert ist, um die Feststoffe innerhalb des Aufnahmetrichters (1) oder zu einem äußeren Kollektor und die Flüssigkeiten durch Pumpen (137) zu mindestens einem Flüssigkeitsablaufspeichertank (18) zu führen, der innen einen Aktivkohlefilter (112) zum Reinigen der Flüssigkeiten hat, während die Sprühdüsen (113) so angeordnet sind, dass sie die Flüssigkeiten durch mindestens einen zusätzlichen Filter (65) und einen geeigneten Schlauch in den Aufnahmetrichter (1), den Niederfrequenzpulsator (39), den Hochfrequenzpulsator (68) und die Stabilisierungskammer (83) sprühen.

11. Ein System zur Aufbereitung von Hausmüll nach den Ansprüchen 1 bis 10, **dadurch gekennzeichnet, dass** die spezielle Öffnungseinheit (19), die spezielle Homogenisierungseinheit (28), das Metallgehäuse (55) des Niederfrequenzpulsators (39), der zweite Kettenförderer (66), das Metallgehäuse (85) des Hochfrequenzpulsators (68), der A(52) und der spezielle Sortierer von recycelbare Materialien (122) Schutzdeckel (25) und Zugangsklappen (26, 53, 56, 80, 86, 151), Fenster (27, 54, 57, 80, 87), visuelle Kontrollsysteme (20) und Wasch- und Sterilisationsanordnungen (130) haben.

## Revendications

1. Un système pour de traitement de Déchets Solides Municipaux, comprenant en entonnoir de réception (1) pour l'entrée des Déchets Solides Municipaux dans le système, sous lequel se trouve un premier convoyeur pour transférer les déchets vers une Unité Spéciale d'Ouverture (19) d'emballages de Déchets Solides Municipaux et une Unité Spéciale d'Homogénéisation (28) des Déchets Solides Municipaux, tandis qu'en dehors de l'entonnoir de réception (1) il y a au moins un système de séparation (17); un élévateur vertical (31); un Doseur Horizontal pour contrôler le flux d'alimentation vers un Pulsateur à Basse Fréquence (39) pour la pulvérisation de matériau; un Pulsateur à Haute Fréquence (68); un Système de Transfert, configuré pour transférer le produit depuis le Pulsateur à Haute Fréquence (68) vers une Chambre de Stabilisation (83), où le Pulsateur à Haute Fréquence (68) est configuré pour produire des pulsations d'une fréquence supérieure à celle du Pulsateur à Basse Fréquence (39), **caractérisé en ce que**:
i) le premier convoyeur est un premier convoyeur a chaîne (5), qui se trouve en contrebas de l'entonnoir de réception (1), de manière à ce qu'en dessous du premier convoyeur a chaîne (5) et le long de l'entonnoir de réception (1) se trouve un réservoir de drainage (15) avec au moins une pompe (16) à fluides épais;
ii) L'Unité Spéciale d'Ouverture (19), qui comprend au moins : un moteur (23), un réseau de poulies et de courroies (24) ou un électro-réducteur et un axe de rotation (21), parallèle au premier convoyeur à chaîne (5) et situé à une distance régulée par rapport à ce-dernier, qui porte des disques de coupe (22) faits dans un matériau résistant, avec une vitesse de rotation régulée et situés à distance du premier convoyeur à chaîne (5);
iii) L'Unité Spéciale d'Homogénéisation (28), qui comprend au moins: un moteur électrique (36) et des réseaux de poulies et de courroies ou un réducteur électrique (79), un axe de rotation (29) qui est horizontal et parallèle au niveau du premier convoyeur a chaîne (5) et situé à une distance régulée par rapport à ce-dernier et porte des plaques métalliques (30), des roulements (35) et un axe de support horizontal (33), parallèle à l'axe de rotation (29), qui est horizontal et parallèle à l'axe de rotation (29), tandis que des supports latéraux (34) sont disposés verticalement des deux côtés de l'axe de support horizontal (33), à l'intérieur de guides verticaux (37) et des ressorts hélicoïdaux (32), verticaux par rapport à l'axe de support horizontal (33), permettant le déplacement vertical de l'Unité Spéciale d'Homogénéisation (28);
iv) L'élévateur vertical (31) a une entrée et une sortie (48), de manière à ce que l'entrée est connectée soit au premier convoyeur a chaîne (5), soit à un convoyeur horizontal (136), tandis que la sortie (48) est connectée à l'entrée (47) du Doseur à Mesure Horizontal (38);
v) Le Doseur à Mesure Horizontal (38) comprend : un cadre métallique (42), un couvercle (44), un fond (45), un tapis roulant (43), des coussinets (51) d'une longueur égale à la largeur du tapis roulant (43) et d'une hauteur telle qu'il n'y a pas d'espace entre le fond (45) et les coussinets (51) et deux axes de rotation (40, 41), où l'un des axes de rotation (40) est mis en rotation par un moteur électrique et un réducteur (134) et est configuré pour actionner le tapis roulant (43), tandis que les coussinets (51) sont configurés pour diriger le produit vers la sortie (49), alors que l'autre axe de rotation (41) est configuré pour tourner librement, tandis que la sortie (49) est reliée avec une entrée (50) du Pulsateur à Basse fréquence (39);
vi) Le Pulsateur à Basse Fréquence (39) situé à l'intérieur d'une structure métallique robuste (55), sur la partie supérieure de laquelle se trouve l'ouverture d'entrée (50), où une sortie d'un entonnoir d'alimentation (52) est connecté et une ouverture de sortie (67) sur la partie basse, comprend: un axe de rotation (58), configuré pour venir verticalement depuis le centre d'au moins deux disques de support métalliques (59), qui supportent des éléments d'impulsion (61) entre eux, attachés avec un axe de rotation perforé (62) sur des axes cylindriques (60), parallèles à l'axe de rotation (58), tandis qu'en partie basse de la structure métallique (55) il y a un réseau métallique d'éléments de contrôle horizontaux (63) du volume, qui sont situés à des distances déterminées entre eux, configurées de manière à créer des ouvertures (64), avec l'ouverture de sortie (67) du matériau vers un second convoyeur à chaîne (66), connecté au Pulsateur à Haute Fréquence (68);
vii) Le Pulsateur à Haute Fréquence (68), situé à l'intérieur d'une structure métallique robuste (85), comprend un axe de rotation (88), configuré pour venir verticalement depuis le centre d'au moins deux disques de support métalliques (89) qui sont parallèles entre eux et verticaux par rapport à l'axe de rotation (88) et supportent entre eux des éléments d'impulsion (91), attachés à une distance déterminée par un axe perforé (92) sur des axes cylindriques (90) qui sont parallèles à l'axe de rotation (88), tandis que le nombre d'axes cylindriques (90) et le nombre d'éléments d'impulsion (91) de chaque axe cylindrique (90) est supérieur ou égal au nombre des éléments d'impulsion (62) et d'axes cylindriques (60) du Pulsateur à Basse Fréquence (39), et tandis qu'en partie basse de la structure métallique (85) il y a un réseau d'éléments de contrôle (93) du Pulsateur à Basse Fréquence (39), et tandis qu'en partie basse de la structure métallique (85) ii y a un reseau d'éléments de contrôle (93) du volume, avec des ouvertures (94) de dimensions plus petites que les ouvertures (64) du réseau métallique d'éléments de contrôle horizontaux (63) du Pulsateur à Basse Fréquence (39);
viii) Le système de Transfert est un système de Transfert d'Air, qui comprend : un réseau de filtres (84), une seconde pompe centrifuge à air (96) de haute pression et sous-pression avec une entrée (97) connectée à la sortie du Pulsateur à Basse Fréquence (68) à travers un tube (98) et une sortie (100), qui est connectée par l'intermédiaire de tubes (101) avec la partie arrière de la Chambre de Stabilisation (83), où la seconde pompe centrifuge à air (96) est configurée pour créer un courent d'air intense dirigé à partir du Pulsateur à haute Fréquence (68) vers la Chambre de Stabilisation (83), provoquant le transfert violent de produit vers ce-dernier et une première pompe centrifuge à air (81) de pression et sous-pression, reliée à la Chambre de Stabilisation (83) et configurée pour en retirer le surplus d'air et d'humidité crée à l'intérieur de la Chambre de Stabilisation (83) à travers un réseau de filtres (84);
ix) la Chambre de Stabilisation (83) est de forme cylindrique, avec un diamètre stable, ancré, avec des ouvertures (117) en partie basse et une sortie (102), qui inclut une partie conique (132) et une partie cylindrique (133), avec un diamètre stable, tandis que la Chambre de Stabilisation (83) comprend : disposés sur la circonférence des parois de la Chambre de Stabilisation (83), mais aussi sur un axe (105), des réseaux émetteurs de radiation UV (114), configurés pour émettre une radiation dans le spectre de 253,7nm, mais aussi dans le spectre de 185nm, de manière à ce que les émetteurs disposés sur l'axe (105) sont recouverts par un matériau transparent spécial pour les protéger, une vis (103) et un agitateur (104), placés axialement par rapport centre de la section verticale de la Chambre de Stabilisation (83), où l'agitateur (104) est de longueur égale à la longueur de la Chambre de Stabilisation (83), dont la section stable maximale est configurée pour avoir une portée atteignant le début de la partie conique (132) de la sortie (102) de la Chambre de Stabilisation (83), tandis que la vis (103) est disposée de manière longitudinale par rapport à la Chambre de Stabilisation (83) et est d'un diamètre inférieur à celui de l'agitateur (104) et d'une longueur plus importante, configurée pour atteindre la partie cylindrique (133) de la sortie (102) de la Chambre de Stabilisation (83), la vis (103) et l'agitateur (104) sont disposés de manière coaxiale sur l'axe (105), isolés des autres parties de la Chambre de Stabilisation (83), où la vis (103) est à l'intérieur de l'agitateur (104) et a un enroulement opposé en relation à l'axe (105) par rapport à l'enroulement se l'agitateur (104), où l'axe (105) a une extrémité libre, tandis que l'autre porte une poulie (107), qui est reliée par l'intermédiaire de courroies (109) faites dans un matériau électriquement isolant à une seconde poulie (109), qui peut être tournée par un moteur (110), la vis (103) et l'agitateur (104) sont en contact électrique avec l'axe (105) et l'agitateur (104) a à ses bouts du matériau isolant (111), qui est en contact avec le corps de la Chambre de Stabilisation (83) et est configuré pour racler, pendant les rotations, les parois intérieures de la Chambre de Stabilisation (83), construite dans un matériau conducteur et le corps de la Chambre de Stabilisation (83) est connecté à mise è terre de protection, tandis que l'axe (105), la vis (103) et l'agitateur (104) sont connectés à une charge positive d'un voltage élevé et d'une tension contrôlée, tandis qu'au moins une pompe (137) est configurée de manière à transférer les liquides traités à partir du réservoir de drainage (15) vers l'intérieur de la Chambre de Stabilisation (83) par l'intermédiaire de buses de pulvérisation (113).

2. Un système pour de traitement de Déchets Solides Municipaux, conformément à la revendication 1, **caractérisé par** ce que l'axe (41) du Doseur à Mesure Horizontal (38) au-dessus de la sortie (49), porte des éléments magnétiques (140) pour la sélection de métaux ferreux, pour ce faire, au fond (45) du Doseur à Mesure Horizontal (38) est située une seconde Sortie (139) pour le transfert des métaux ferreux sélectionnés vers une collecte séparée.

3. Ln système pour de traitement de Déchets Solides Municipaux, conformément aux revendications i et 2, **caractérisé en ce que**, en partie basse de la Chambre de Stabilisation (83), disposés à intervalles réguliers, se situent des ouvertures (117), chaque ouverture (117) possédant un filtre métallique (120, 121 et 141) avec des trous, où le premier filtre métallique (120) a les trous les plus petits, le second filtre métallique (121) a des trous plus larges et le troisième filtre métallique a les trous les plus larges et la Chambre de Stabilisation (83) porte en son intérieur une seconde vis (118) avec un pas de direction opposée à partir des bords vers le centre, qui est configuré pour conduire le matériau à partir des bords vers le centre, tandis que la partie basse de la seconde vis (118) possède trois ouvertures (119) pour la sélection finale d'éléments.

4. Un système pour de traitement de Déchets Solides Municipaux, conformément aux revendications 1 à 3, **caractérisé en ce qu'**un Trieur Spécial de Matériaux Recyclables (122) est configuré afin d'être utilisé pour la séparation de verre, d'éléments de métaux ferreux, d'aluminium et de plastique et est connecté à l'ouverture centrale (119), où à l'intérieur du Trieur Spécial de Matériaux Recyclables (122) se trouve un tambour à rotation (123) avec des éléments magnétiques (142), un fût de raclage spécial (124) qui est en contact tangent avec le tambour à rotation (123), un collecteur (125) pour les éléments de métaux ferreux et une pompe centrifuge (144), configurée pour créer un courant d'air et de guider les éléments légers vers le tambour électrostatique en rotation (146), avec un réseau métallique se raclage (148) pour détacher le plastique, qui est en contact langent avec le tambour électrostatique en rotation (146), sous un angle réglé au travers de régulateurs tournants externes (150), avec un collecteur (127) pour les plastiques, un collecteur (126) pour l'aluminium et un collecteur (128) pour le verre.

5. Un système pour de traitement de Déchets Solides Municipaux, conformément aux revendications 1 à 4, **caractérisé en ce qu'**une Chambre à Micro-ondes (116) avec des réseaux d'émission de micro-ondes (115) est située à la sortie de la Chambre de Stabilisation (83).

6. Un système pour de traitement de Déchets Solides Municipaux, conformément aux revendications 1 à 5, **caractérisé en ce qu'**un Réseau Final de Stabilisation (131) pour la Réduction du Volume et de la Masse est connecté à la Chambre à Micro-ondes (116).

7. Un système pour de traitement de Déchets Solides Municipaux, conformément aux revendications 1 à 6, **caractérisé en ce que** le second convoyeur à chaîne (66) est du type clos et possède un axe de rotation (69), (70) à ses deux extrémités, où l'axe de rotation (69), situé en dessous du Pulsateur à Basse Fréquence (39), est connecté à un moteur électrique (71) par l'intermédiaire d'un réducteur (72), configuré pour transférer le mouvement vers une chaîne (73) du second convoyeur à chaîne (66), tandis que l'axe de rotation (70) situé au-dessus du Pulsateur à Haute Fréquence (68), est placé dans des guides (74), qui sont configurés pour permettre le mouvement horizontal relatif au niveau de mouvement du second convoyeur à chaîne (66) et chaque axe de rotation (69), (70) possède à chaque bout des disques à crans (75), verticaux par rapport aux bords des axes de rotation (69), (70), qui sont d'un même nombre que les chaînes (73) du convoyeur à chaîne (66), où chaque disque à crans (75) possède sur sa surface des creux, dans lesquels chaque chaîne (73) est configurée pour reposer et des feuilles métalliques (77) sont placées verticalement par rapport aux chaînes (73) à des intervalles réguliers et à une distance entre elles variant entre 40 cm et 80 cm environ, où sur chaque feuille métallique (77) et sur l'intérieur du second convoyeur à chaîne (66) est disposée une bande longitudinale de matériau (78) d'une longueur égale à la longueur de la feuille métallique (77), où la seconde pompe centrifuge (96) crée un courent d'air artificiel violent à travers le second convoyeur à chaîne (66).

8. Un système pour de traitement de Déchets Solides Municipaux, conformément aux revendications 1 à 7, **caractérisé en ce que** l'entonnoir de réception (1) est doté de feuilles pare-vent (2), une photocellule (4) pour le contrôle du niveau de matériel, un système de blocage des déchets (138) et un regard de visite (3).

9. Un système pour de traitement de Déchets Solides Municipaux, conformément aux revendications 1 à 8, **caractérisé en ce que** le premier convoyeur à chaîne (5) est équipé d'un système de contrôle du flux, afin d'éviter les blocages dans l'Unité Spéciale d'Ouverture (19), avec une vitesse du mouvement régulée et comprenant au moins une chaîne (6) et une surface de glissement (7) qui est compacte et est en pente ou est perforée et métallique et porte sur les deux côtés de ses bords deux axes cylindriques (8) avec des disques crantés (9) dont le nombre est égal au nombre de chaînes (6), où, au sein de leur périmètre, les disques crantés (9) ont des creux (10) de taille adéquate, dans lesquels chaque chaîne (6) st posée, où le premier convoyeur à chaîne est prolongé sous l'unité spéciale d'homogénéisation (28) et est configuré pour déposer le matériau transféré directement vers l'entrée de l'élévateur vertical (31) ou vers le convoyeur horizontal (136).

10. Un système pour de traitement de Déchets Solides Municipaux, conformément aux revendications 1 à 9, **caractérisé en ce que** la pompe (16) pour fluides épais se trouve à l'intérieur du réservoir de drainage (15) et est configurée pour mener les fluides épais à partir du réservoir de drainage (15) vers le système de séparation (17), qui est configuré pour amener las solides vers l'intérieur de l'entonnoir de réception (1) ou vers un collecteur extérieur et les fluides à travers des pompes (137) vers au moins un réservoir de stockage du drain liquide (18), qui comporte en son intérieur un filtre au carbone actif (112) pour la purification des fluides, tandis que des buses de pulvérisation (113) sont arrangées pour pulvériser les liquides, à traves au moins un filtre supplémentaire (65) et un tuyautage adéquat, dans l'entonnoir de réception (1), le Pulsateur à Basse Fréquence (39), le Pulsateur à Haute Fréquence (68) et la Chambre de Stabilisation (83).

11. Un système pour de traitement de Déchets Solides Municipaux, conformément aux revendications 1 à 10, **caractérisé en ce que** l'Unité Spéciale d'Ouverture (19), l'Unité Spéciale d'Homogénéisation (28), la structure métallique (55) du Pulsateur à Basse Fréquence (39), le second convoyeur à chaîne (66), la structure métallique (85) du Pulsateur à Haute Fréquence (68), entonnoir d'alimentation (52) et le Trieur Spécial de Matériaux Recyclables (122) ont des couvercles de protection (25), des trappes d'accès (26, 53, 56, 80, 86, 151), des fenêtres (27, 54, 57, 80, 87), des systèmes de contrôle visuel (20) et des réseaux de lavage et de stérilisation (130).
